(19) Europäisches Patentamt
European Patent Office
Office européen des brevets

(11) EP 1 587 839 B1

(12) **EUROPEAN PATENT SPECIFICATION**

(45) Date of publication and mention of the grant of the patent:
**08.10.2008 Bulletin 2008/41**

(21) Application number: **04704181.9**

(22) Date of filing: **22.01.2004**

(51) Int Cl.:
***C07K 17/00*** *(2006.01)* ***G01N 33/68*** *(2006.01)*
***C12N 11/00*** *(2006.01)*

(86) International application number:
**PCT/DK2004/000047**

(87) International publication number:
**WO 2004/065928 (05.08.2004 Gazette 2004/32)**

(54) **LIGHT INDUCED IMMOBILISATION**

LICHT-INDUZIERTE IMMOBILISIERUNG

IMMOBILISATION PHOTOGENEREE

(84) Designated Contracting States:
**AT BE BG CH CY CZ DE DK EE ES FI FR GB GR HU IE IT LI LU MC NL PT RO SE SI SK TR**

(30) Priority: **22.01.2003 DK 200300081**
**22.01.2003 US 441975 P**

(43) Date of publication of application:
**26.10.2005 Bulletin 2005/43**

(73) Proprietor: **Bionanophotonics A/S**
**1260 Copenhagen K (DK)**

(72) Inventors:
- **PETERSEN, Steffen, Björn**
  **DK-9200 Aalborg (DK)**
- **DA CRUZ AUGUSTO NEVES PETERSEN, Maria, Teresa**
  **DK-9200 Aalborg (DK)**

(74) Representative: **Nilausen, Kim et al**
**Zacco Denmark A/S**
**Hans Bekkevolds Allé 7**
**2900 Hellerup (DK)**

(56) References cited:
**WO-A-91/16425**
**WO-A-94/01773**
**US-A- 3 959 078**
**US-A- 5 412 087**
**US-B1- 6 350 368**

- **COLLIOUD A ET AL: "ORIENTED AND COVALENT IMMOBILIZATION OF TARGET MOLECULES TO SOLID SUPPORTS: SYNTHESIS AND APPLICATION OF A LIGHT-ACTIVATABLE AND THIOL-REACTIVE CROSS-LINKING REAGENT" BIOCONJUGATE CHEMISTRY, AMERICAN CHEMICAL SOCIETY, WASHINGTON, US, vol. 4, no. 6, 1993, pages 528-536, XP001121631 ISSN: 1043-1802 cited in the application**
- **NEVES-PETERSEN MT ET AL.: "High probability of disrupting a disulphide bridge mediated by an endogenous excited tryptophan residue" PROTEIN SCIENCE, vol. 11, 2002, pages 588-600, XP002288112 cited in the application**
- **DOSE K: "The Photolysis of Free Cystine in the Presence of Aromatic Amino Acids" PHOTOCHEMISTRY AND PHOTOBIOLOGY, vol. 8, 1968, pages 331-335, XP008032773**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 587 839 B1

Printed by Jouve, 75001 PARIS (FR)

## Description

Field of the invention

The present invention relates to a method of cross-linking or immobilising proteins on a carrier.

Background of the invention

Molecules can be immobilised on a carrier or solid surface either passively through hydrophobic or ionic interactions, or covalently by attachment to activated surface groups. In response to the enormous importance of immobilisation for solid phase chemistry and biological screening, the analytical uses of the technology have been widely explored. The technology has found broad application in many different areas of biotechnology, e.g. diagnostics, biosensors, affinity chromatography and immobilisation of molecules in ELISA assays. The value of immobilisation technology is demonstrated by the recent development of DNA microarrays, where multiple oligonucleotide or cDNA samples are immobilised on a solid surface in a spacially addressable manner. These arrays have revolutionised genetic studies by facilitating the global analysis of gene expression in living organisms. Similar approaches have been developed for protein analysis, where as little as one picogram of protein need be bound to each point on a microarray for subsequent analysis. The proteins bound to the microarrays, can then be assayed for functional or structural properties, facilitating screening on a scale, and with a speed, previously unknown. The biomolecules bound to the solid surface may additionally be used to capture other unbound molecules present in mixture.

Development of this technology, with the goal of immobilising a biomolecule on a solid surface in a controlled manner, with minimal surface migration of the bound moiety and with full retention of its native structure and function, has been the subject of intensive investigation in recent years (Veilleux J (1996) IVD Technology, March p. 26-31). The simplest type of protein immobilisation exploits the high inherent binding afinity of surfaces to proteins in general. For example proteins will physically adsorb to hydrophobic substrates via numerous weak contacts, comprising van der Waals, and hydrogen bonding interactions. The advantage of this method is that it avoids modification of the protein to be bound. On the other hand, proteins bound in this manner may be distributed unevenly over the solid support and/or inactivated since, for example, their clustering may lead to steric hindrance of the active site/binding region in any subsequent functional assay.

Alternative methods of immobilisation rely on the use of a few strong covalent bonds to bind the protein to the solid surface (Wilson D.S., Nock S., 2001, Current Opinion in Chemical Biology 6:81-85). Examples include immobilisation of biotinylated proteins onto streptavidin-coated supports, and immobilisation of His-tagged proteins, containing a polyhistidine sequence, to $Ni^{2+}$-chelating supports. Other functional groups on the surface of proteins which can be used for attachment to an appropriate surface include reacting an amine with an aldehyde via a Schiff-base, cross-linking amine groups to an amine surface with gluteraldehyde to form peptide bonds, cross-linking carboxylic acid groups present on the protein and support surface with carbodiimide, cross-linking based on disulfide bridge formation between two thiol groups and the formation of a thiol-Au bond between a thiol group and a gold surface.

Amine coupling is a widely used method of immobilisation chemistry. N-hydroxysuccinimide esters are formed from a fraction of the carboxyl groups of the carboxymethyldextran matrix via reaction with N-hydroxysuccinimide (NHS) and N-ethyl-N'-(dimethylaminopropyl) carbodiimide hydrochloride (EDC) in water, which then react spontaneously with amine groups on a protein to form covalent bonds (Johnsson B., et. al., 1991, Anal Biochem 198:268-77). Following immobilisation, un-reacted N-hydroxysuccinimide esters on the support are deactivated with 1 M ethanolamine hydrochloride to block areas devoid of bound proteins. The method is laborious since the reagents, used at each step of a chemical immobilization method, usually need to be removed prior to initiating the next step.

Methods for the immobilization of biomolecules via disulfide bridges are described by Veilleux J (1996) *supra.* Protein samples are treated with a mild reducing agent, such as dithiothreitol, 2-mercaptoethanol or tris(2-carboxyethyl) phosphine hydrochloride to reduce disulfide bonds between cysteine residues, which are then bound to a support surface coated with maleimide. Alternatively primary amine groups on the protein can be modified with 2-iminothiolane hydrochloride (Traut's reagent) to introduce novel sulfhydryl groups, which are thereafter immobilized to the maleimide surface. Immobilization of proteins on a gold substrate via a disulfide bridge is shown for the cupredoxin protein plastocyanin from Poplar (Andolfi, L. et al. 2002, Arch. Biochem. Biophys. 399: 81-88). Since this protein lacks a disulfide bridge, surface exposed residues Ile21 and Glu25 were both substituted with Cys. Disulfide bridge formation between the inserted cysteines was confirmed from the 3D crystal structure of the purified mutant plastocyanin. Mutant plastocyanin, expressed intracellularly in bacteria, is exposed to a reducing environment in the cytoplasm, such that the inserted cysteines are reduced, and can thus mediate the direct adsorption of the isolated protein onto a gold substrate. The thiol group binding properties of the protein are thus dependent on *in vivo* or *in vitro* chemical reduction of the cysteine residues on the surface of the protein.

An alternative approach to engineering thiol-group binding properties into a protein has been described for ribonuclease (RnaseA), which has four essential cystines (Sweeney, R.Y. et al. 2000 Anal Biochem. 286: 312-314). In this case a single cysteine residue was substituted for Ala19, located in a surface loop near the N-terminus of RNase A. The cysteine in the expressed RNase was protected as a mixed disulfide with 2-nitro-5-thiobenzoic acid. Following subsequent de-protection with an excess of dithiothreitol, the RNase was coupled to the iodoacetyl groups attached to a cross-linked agarose resin, without loss of enzymatic activity. Again, preparation of the protein for immobilisation requires its exposure to both protecting and deprotecting agents, which may negatively impact its native structure and/or function.

Methods are described in US 6,350,368 for the coupling of FAD-dependent enzymes onto electrodes, whereby an apoenzyme is complexed with a functionalised FAD that is covalently bonded to a binding moiety, capable of chemical association (e.g. through a thiol group) or attachment on the surface of an electrode (e.g. gold). The immobilised FAD-enzyme complex is further functionalised with an electron mediator group and can be used in electrochemical systems for deforming analytes or optical signals.

Light-induced immobilization techniques have also been explored, leading to the use of quinone compounds for photochemical linking to a carbon-containing support (EP0820483). Activation occurs following irradiation with non-ionising electromagnetic radiation in the range from UV to visible light. Masks can be used to activate certain areas of the support for subsequent attachment of biomolecules. Following illumination the photochemically active compound, anthraquinone, will react as a free radical and form a stable ether bond with a polymer surface. Since anthraquinone is not found in native biomolecules, appropriate ligands have to be introduced into the biomolecule. In the case of proteins, this additional sample preparation step may require thermochemical coupling to the quinone and may not be site specific.

A further development of light-induced immobilisation technology is disclosed in US 5,412,087 and US 6406844, which describe a method for preparing a linker bound to a substrate. The terminal end of the linker molecule is provided with a reactive functional group protected with a photo-removable protective group, e.g. a nitro-aromatic compound. Following exposure to light, the protective group is lost and the linker can react with a monomer, such as an amino acid at its amino or carboxy-terminus. The monomer, furthermore, may itself carry a similar photo-removable protective group, which can also be displaced by light during a subsequent reaction cycle. The method has particular application to solid phase synthesis, but does not facilitate orientated binding of proteins to a support.

Bifunctional agents possessing thermochemical and photochemical functional substituents for immobilising an enzyme are disclosed in US 3,959,078. Derivatives of arylazides are described which allow light mediated activation and covalent couping of the azide group to an enzyme, and substituents which react thermochemically with a solid support. The orientation of the enzyme molecules immobilised by this procedure is not controlled.

A method for orientated, light-dependent, covalent immobilization of proteins on a solid support, using the heterobifunctional wetting-agent N-[m-[3-(trifluoromethyl)diazirin-3-yl]phenyl]-4-maleimidobutyramine, is described in WO 91 16425 and by Collioud A et al. (1993) in Bioconjugate Chem. 4: 528-536. The aryldiazirine function of this cross-linking reagent facilitates light-dependent, carbene-mediated, covalent binding to either inert supports or to biomolecules, such as proteins, carbohydrates and nucleic acids. The maleimide function of the cross-linker allows binding to a thiolated surface by thermochemical modification of cysteine thiols. Orientated binding of this cross-linking reagent to a protein can be attained by a thermochemical interaction between the maleimide function and an exposed thiol group on the protein surface, however this treatment may modify the structure and activity of the target protein. Light-induced covalent coupling of the cross-linking reagent to a protein via the carbene function, however, has the disadvantage that it does not provide for controlled orientation of the target protein.

WO 94/01773 discloses photoactivation of proteins by ultraviolet radiation, which enables reaction with other chemical entities to form conjugates.

Common for most of the described immobilisation methods is their use of one or more thermochemical/chemical steps, sometimes with hazardous chemicals, some of which are likely to have a deleterious effect on the structure and/or function of the bound protein. The available methods are often invasive, whereby foreign groups are introduced into a protein to act as functional groups, which cause protein denaturation, as well as lower its biological activity and substrate specificity.

There is a need in the art of protein coupling and immobilisation to improve the method of coupling, where the structural and functional properties of the coupled or immobilised component are preserved and the orientation of coupling can be controlled.

## Summary of invention

The present invention provides a method for coupling a protein or a peptide on a carrier via stable bonds (covalent bond or thiol-Au bond) while preserving the native structural and functional properties of the coupled protein or peptide, and avoiding the use of one or more chemical steps. This contrasts with traditional coupling methods for protein immobilisation, which typically involve several, chemical reactions, which can be costly, time-consuming as well as deleterious to the structure/function of the bound protein. Furthermore the orientation of the protein or peptide, coupled according to the method of the present invention, can be controlled, such that their functional properties e.g. enzymatic, may be preserved. In comparison, the majority of known protein coupling methods lead to a random orientation of the proteins immobilised on a carrier, with the significant risk of lower biological activity and raised detection limits. The invention exploits an inherent property of proteins and peptides, whereby a disulfide bridge in a protein or peptide, located in close proximity to an aromatic amino acid residue, is disrupted following prolonged irradiation with light absorbed by these aromatic amino acids. The thiol groups created by light-induced disulfide bridge disruption in a protein or peptide are then used to immobilise the protein or peptide to a carrier.

According to a first aspect of the invention, a method of coupling a disulfide bridge containing protein or peptide on a carrier is provided, comprising the following steps of irradiating a protein or peptide creating a thiol group, and incubating the irradiated protein or peptide with a carrier capable of binding a thiol group, wherein the carrier is a support whereon more than one disulfide-bridge-containing protein or peptide are coupled. Coupling according to the invention is furthermore possible by incubating the protein or peptide together with a carrier capable of binding a thiol group and irradiating the protein or peptide in the presence of the carrier to create a thiol group in the protein or peptide, and thereby obtaining a coupling to the thiol ligand binding group on the carrier. According to this method of coupling or immobilization, the irradiation comprises light of a wavelength that excites one or more aromatic amino acids. The irradiated aromatic amino acid in the protein may comprise tryptophan, tyrosine and phenylalanine, or alternatively the aromatic amino acid is tryptophan. According to a further aspect of the invention, the irradiation comprises wavelengths in the vicinity of 295nm, 254nm or 275nm. According to a further aspect of the invention, the irradiated protein or peptide is coupled to a carrier that comprises a peptide or protein or any other biomolecule. Alternatively the irradiated protein or peptide is coupled to a support which comprises gold, or is derivatised with a thiol binding group, or comprises a thiol group or a disulfide bridge, where coupling to the support may be an immobilisation. Optionally the carrier or support comprises a spacer. According to a further aspect of the invention is provided a carrier or support comprising the coupled protein or peptide, produced according to the disclosed method of coupling a protein or peptide on a carrier. According to a further aspect of the invention, the protein or peptide, coupled according to the disclosed method of coupling a protein or peptide on a carrier includes enzymes, transcription factors, protein domains, antigens, antibodies or binding proteins as well as other proteins or peptides. According to a further aspect of the invention the carrier, coupled according to the disclosed method of coupling a protein or peptide on a carrier, includes a pharmaceutical drug. According to a further aspect of the invention is the use of the carrier or support comprising the coupled protein, produced according to the disclosed method of coupling a protein or peptide on a carrier, for bio-functional assays or in a kit providing for biofunctional assays. This use for bio-functional assays comprises: bio-sensors, chromatography, immunodetection, enzyme assays, nucleotide binding detection, protein-protein interaction, protein modifications, carrier targeting or protein targeting.

Brief description of drawings

Figure 1 shows the cutinase fluorescence emission intensity at 350 nm as a function of time of illumination at 295 nm.

Figure 2 shows the coupling reaction between an SH group of a protein and an SS group of 5,5'-dithiobis-(2-nitrobenzoic acid) (DTNB) and the stoichiometric release of nitrothiobenzoate (NTB).

Figure 3 shows the Trp fluorescence (F) emission intensity increase of cutinase at 350 nm [F/Fo] as a function of time of illumination at 295 nm (solid line) and the concentration of newly formed free thiol groups in cutinase samples with specific ratios of fluorescence emission increase [F/Fo] (open circles).

Figure 4 shows the fluorescence emission spectrum of a 1μM cutinase solution upon excitation with 295 nm light during incubation (A) at 25˚C without DTT [solid circles]; (B) at 70˚C without DTT [solid black line]; (C) at 25˚C without DTT, following heating to 70˚C and cooling to 25˚C [hollow circles]; (D) at 25˚C with DTT, following heating to 70˚C, addition of DTT and cooling to 25˚C [solid grey line].

Figure 5 shows spectral characteristics of aromatic amino acid residues in aqueous solution at pH 7.0.

Figure 6 shows the structure of the intramolecularly quenched probe Bodipy FL- L-Cystine, whose SS bond break in the presence of free SH groups, resulting in fluorescence of the Bodipy groups.

Figure 7 shows detection of free SH groups in a protein sample before and after UV illumination at 296nm by reaction with Bodipy FL- L-Cystine. The Bodipy group fluorescence emission spectrum of mutant cutinase (W69A) (A); native cutinase (B) protein samples is shown for: non-illuminated and minus Bodipy probe ( ); non-illuminated with Bodipy probe (•) and illuminated with bodipy (•) treatments.

Figure 8 shows detection of free SH groups in a protein sample before and after UV illumination at 296nm by reaction with Bodipy FL- L-Cystine. The Bodipy group fluorescence emission spectrum of hen white lysosyme (A); *Rhizopus niveus* triglyceride lipase (B); human plasminogen (C); human placental alkaline phosphatase (D); chymosin (E); human immunoglobulin (F) protein samples is shown for: non-illuminated and minus Bodipy probe; non-illuminated with Bodipy probe; and illuminated with bodipy treatments.

Figure 9 shows the contour plots of different spatial Trp S-S triad neighbourhood spheres within a radius of 8Å. The different triads are plotted along the x-axis, and ranked according to their similarity to the cutinase triad (1cex; ranked No.1) and the different groups of residues are plotted along the y-axis. A score of >1 means that a residue/group is over-represented, while a score of <1 means that a residue/group is under-represented.

Figure 10 shows the contour plots of different spatial Trp S-S triad neighbourhood spheres within a radius of 8Å. The different triads are plotted along the x-axis, and ranked according to their similarity to the cutinase triad (1cex; ranked No.1) and the different groups of residues are plotted along the y-axis. A score of >1 means that a residue/group is over-represented, while a score of <1 means that a residue/group is under-represented. Two Trp S-S triads in α-lactalbumin are included in the analysis, where Trp60-Cys73-Cys91 is found to be disruptable on UV irradiation, while Trp118-Cys28-Cys111 is not disruptable.

Figure 11 shows the contour plots of different spatial Trp S-S triad neighbourhood spheres within a radius of 8Å identified in IGG1 triads A present in immunoglobulins. The different triads are plotted along the x-axis, and ranked according to their similarity to the cutinase triad (1cex; ranked No.1) and the different groups of residues are plotted along the y-axis. A score of >1 means that a residue/group is over-represented, while a score of <1 means that a residue/group is under-represented.

Figure 12 shows the contour plots of different spatial Trp S-S triad neighbourhood spheres within a radius of 8Å identified in IGG1 triads B present in immunoglobulins. The different triads are plotted along the x-axis, and ranked according to their similarity to the cutinase triad (1 cex; ranked No.1) and the different groups of residues are plotted along the y-axis. A score of >1 means that a residue/group is over-represented, while a score of <1 means that a residue/group is under-represented.

Figure 13 shows a time course of fluorescence emission at 330nm, on excitation at 296nm, detected in a TIRF flow cell during cutinase immobilisation on a SH-activated quartz slide under continuous or limited illumination at 296nm. The flow chamber in step A: is flushed with 2.0 μM cutinase solution; step B: incubated with 2.0 μM cutinase solution; step C/D: rinsed with buffer A; and step E: rinsed with detergent and buffer A.

Figure 14 A: shows the final fluorescence emission intensity (at 330nm, on excitation at 296nm) of native cutinase immobilised on a SH-activated or a hydroxylated quartz slide, under continuous or limited illumination. B: shows the final fluorescence emission intensity (at 330nm, on excitation at 296nm) of native cutinase and mutant cutinase (W69A) immobilised on a SH-activated quartz slide, under continuous illumination or in the dark.

Figure 15 shows fluorescence emission intensity (at 450nm, on excitation at 365nm) of 4-methylum-belliferylbutyrate hydrolysed by cutinase immobilised on either SH-activated or hydroxylated quartz slides by UV-illumination.

Figure 16 shows a time course of fluorescence emission at 330nm, on excitation at 296nm, detected in a TIRF flow cell during (A) lysozyme (B) lysosyme and (C) chymosin immobilisation on a SH-activated or a hydroxylated quartz slide on 10 min continuous illumination (A), or continuous illumination (C) at 296nm, and in the dark (B). In Figure 16 A, B and C the flow chamber in step (a/A) is incubated with the protein (lysosyme or chymosin) 10 minutes (initial 6.66 minutes with flushing/flow); in step (b/B) is washed with buffer for 10 minutes (initial 6.66 minutes with flushing/flow); in step (c/C) is washed with Helmanex, 2% pH 10-11 4-5 minutes (initial 3.33 minutes with flushing/flow); and in step (d/D) is washed with buffer for remaining reading (Initial 3.33 minutes with flow).

Figure 17 A: shows relative activity of a cutinase sample (compared to a sample placed in the dark) as a function of illumination at 296nm or placed in laboratory light. B: shows relative activity of a cutinase sample in the presence of 5.1 mM DTT (•,•), or without DTT (○). The data point at 300 min for the sample without DTT was measured at 1600 min.

## Definitions

As used herein, the terms "UV light" or "irradiation" or UV illumination" or "UV irradiation" is a range of wavelengths or single wavelength of UV light, or IR/visible light for multiphoton excitation, that specifically excites aromatic amino acids, or other aromatic system that may mimic aromatic amino acids, preferably a wavelength of approximately 295 nm, 275 nm or 254 nm, more preferably the wavelengths that excite tryptophan, tyrosine or phenylalanine, most preferably the wavelength, 295 nm, that excites tryptophan.

As used herein the term "protein" comprises polypeptides, fragments of proteins and antibodies or any other amino acid based material. Furthermore the term "protein" includes enzymes, antibodies, antigens, transcription factors, binding proteins e.g. DNA binding proteins, or protein domains.

As used herein, the term "spatial neighbour" relates to the physical distance between two chemical groups within a composition, such that groups lying in three-dimensional close proximity are considered to be spatial neighbours. A disulfide bridge in a protein that is a spatial neighbour to an aromatic residue may function as a quencher if the aromatic amino acid absorbs excitation energy following irradiation. The physical distance between half cystines of a disulfide bridge, which are spatial neighbours to tryptophan residues and may act as quenchers, can be, but is not limited, a range of 1 to 10 Å.

As used herein, the term "carrier" can be a soluble compound or polymer, or an insoluble compound or polymer, where the compound or polymer comprises a thiol-binding ligand, such as a reactive SH or an SS bond capable of coupling to an irradiation-induced thiol group. The term carrier can also be another biomolecule, for example another protein, peptide or polypeptide. Furthermore the term "carrier" should be understood to include supports comprising material capable of coupling to an irradiation-induced thiol group, such as a gold support or a derivatised support carrying a thiol-binding ligand.

As used herein, the term "support" can be any support material such as electronic chips, slides, wafers, particles, resins, wells, tubes or membranes which include but are not limited to any material comprising polymers such as Topaz, polystyrene, polyethylene, polyester, polyethermides, polypropylene, polycarbonate, polysulfone, polymethylmethacrylate [PMMA], poly(vinylidene flouride) [PVDF], silicone; diamond; glass e.g. quartz and silica; silicium e.g. silicium wafers; metals; membranes e.g. nylon membranes, nitrocellulose filters; porous materials such as gels, agarose or cellulose; ceramics etc, which furthermore include all forms of derivatisation of the support which facilitate binding of thiol groups with or without intervening spacers.

As used herein, the term "spacer" comprises a chain of compounds with the purpose of providing a link between a protein and a carrier or raising an immobilised protein above the surface of a support.

As used herein, the term 'pharmaceutical drug' comprises articles intended for use in the diagnosis, cure, mitigation, treatment, or prevention of disease in man or other animals; and articles (other than food) intended to affect the structure or any function of the body of man or other animals and articles intended for use as a component of any article specified above.

As used herein the term "bio-functional assay" comprises a biosensor, immunodetection, an enzyme assay, nucleotide-binding detection, chromatography, protein-protein interaction, protein modification, carrier targeting or protein targeting.

As used herein, the term "biosensor" comprises an analytical devise incorporating biological or biologically-derived sensing elements, such as an amino acid (e.g., cysteine), protein, antibody, nucleic acid, microorganism, or cell. The sensing element is either integrated within or intimately associated with a physicochemical transducer. The general aim of a biosensor is to produce either discrete or continuous digital electronic signals that are proportional to a single analyte or a related group of analytes.

## Detailed description of the invention

The present invention exploits an inherent property of proteins, concerning irradiation-induced structural changes in proteins, thought to retard their photo-degradation. When proteins are exposed to UV irradiation some disulfide bridges are disrupted to form activated thiols. Although disulfide bridges are commonly found in the structural core and near/on the surface of folded proteins, those located in close proximity to aromatic amino acids are the most susceptible to UV-induced disruption. During UV exposure of proteins, energy absorbed by side chains of aromatic amino acid residues are transferred to neighbouring disulfide bridges, which function as quenchers (Neves-Petersen MT., et al., 2002, Protein Science 11: 588-600). However, the flow of energy transferred to disulfide bridges and the likely formation of intermediate chemical species such as radicals/ions formed upon light excitation of the sample ultimately serves to trigger their disruption. The presence of a disulfide bridge with tryptophan as a close spatial neighbour in a protein occurs frequently in nature, indicating that photo-induced disulfide bridge disruption is a widespread phenomenon (Petersen MTN., et al., 1999, Protein Engineering 12: 535-548; Neves-Petersen MT et al., 2002, Protein Science 11: 588-600; Vanhooren A et al. 2002, Biochemistry 10; 41(36):11035-11043)."

The exploitation of this irradiation-induced phenomenon for the orientated immobilisation of proteins on a carrier is the basis for this invention. Cutinase, from the fungus *Fusarium solani pisi,* is one of several model proteins that are provided to illustrate the process of light-induced disulfide bridge reduction and their immobilisation on a carrier, however the application of the invention is by no means limited to a model protein. Cutinase is a lipolytic enzyme capable of degrading cutin, an insoluble lipid-polyester matrix found on the surface of plant leaves. Cutinase is an industrially important enzyme, and it is proposed to incorporate in detergents for the removal of fats. It has two disulphide bridges; one near the active site, and one distal at the opposite pole of the protein, in close proximity to the single tryptophan residue of the protein. Chemical reduction of the disulphide bridge, located near the active site, renders the enzyme inactive (Soliday CL, et. al., 1983, Biochem Biophys Res Commun 114:1017-22).

In its native conformation the single tryptophan residue of *F. solani pisi* cutinase is highly quenched due to the presence of the adjacent disulfide bridge. Following prolonged selective irradiation of cutinase at 295nm, the fluorescence quantum yield of the single Trp residue increases simultaneously with the disruption of the neighbouring disulfide bridge. The increased quantum yield of the Trp residue in cutinase reflects the loss of the disulfide bridge and its ability to quench the Trp residue in an excited-state.

Disulphide bridges are known to be excellent quenchers of excited-state aromatic residues. Any aromatic residue, which is in close spatial proximity, can cause photo-induced disruption of a neighbouring disulfide bridge. Hence the three aromatic amino acids, tryptophan, tyrosine and phenylalanine found in proteins, are all potential mediators of light-induced disulfide bridge disruption. While irradiation with light of a range of wavelengths extending from 240nm to 300nm will excite all aromatic residues, the individual aromatic residues have differing absorption maxima (Table 1; data obtained at neutral pH).

Table 1

| In water | Absorption Max. | Emission Max. |
|---|---|---|
| Phe | 254 nm | 282 nm |
| Tyr | 275 nm | 303 nm |
| Trp | 280 nm | 250 nm |

Since the excitation spectrum of the aromatic amino acid residues is only partially overlapping, protein irradiation at a single or narrow wavelength range will excite the individual residues to different degrees. Irradiation at 295nm can be used to selectively excite tryptophan residues in a protein. Irradiation at 280nm will excite both tyrosine and tryptophan residues, which can both then cause photo-induced disulfide bridge disruption. Where irradiation is performed by multiple-photon excitation, for example when two-photon excitation is carried out, the sample is irradiated with photons (light) with half the energy (twice the wavelength) of the photons used in a single-photon experiment. For example, electronic excitation of tryptophan can both be achieved with ultraviolet light at 295 nm, or with two-photon excitation at a wavelength of approximately 690 nm. Furthermore excited tyrosine residues can cause the excitation of neighbouring tryptophan residues by a mechanism called fluorescence resonance energy transfer, which in turn can cause disulfide bridge disruption. It will be apparent to those skilled in the art that the disruption of disulfide bonds in a given protein at a selected wavelength can be predicted from the location and amino acid neighbours of each disulfide bridge in the 3D structure of the protein. Disulphide bridges placed in the spatial vicinity of aromatic amino acid residues are likely to be the most labile to UV light. The 3D structures of a subset of proteins containing the spatial triad Trp Cys-Cys, in close spatial proximity, have been examined in order to identify which amino acids are located in immediate vicinity of the tryptophan residues of the triad. This analysis has identified those proteins having a similar amino acid neighbourhood composition around the triad to that of cutinase, which can be used to predict which proteins will have the disulphide bond of the triad broken upon UV illumination. The features of the amino acid composition around the spatial TrpSS triad in a protein, which may be used to predict whether it will be susceptible to UV-induced disruption, are as follows: 1) an aromatic amino acid residue is located within 10Å of the disulfide bond; 2) those Trp-SS triads in which the angle between the plane of the emitting dipole of the aromatic Trp side chain is orthogonal to the plane of the absorbing dipole of the disulfide bridge are not expected to exchange excitation energy between excited state Trp and a disulfide bridge; 3) a specific subset of amino acids residues are found within an 8Å radius of a UV-disruptable Trp S-S triad of a protein, as demonstrated for cutinase, lysosyme, lipase, plasminogen, chymosin and $\alpha$-lactalbumin, (and immunoglobulin - see below) which are distinct from the average frequency of occurrence of amino acids in a protein in general. In this specific subset of amino acids, the amidic amino acid residues (Asn, Gln) are over-represented, and in the majority of cases the occurrence of short aliphatic residues (Gly, Ala, Val) and/or long aliphatic residues (Leu, Ile) are also over-represented. Comparing the 8 Ångstrøm map to the 12 Ångstrøm and the 15 Ångstrøm maps it can be observed how the contours of the map fade. The amino acid composition of the subsets approaches the composition of the average protein. An observation that can be made on all of the three maps is that in almost every triad there is an under-representation of the groups of charged amino acids (His, Lys, Arg)(Asp, Glu) and proline residues.

The amino acid residues located within an 8Å radius proximity of Trp-SS triads of immunoglobulins, have an over-representation of hydroxyl-containing amino acids, and in many IgG Trp S-S triads the aromatic residues are also over-represented. One or both amidic amino acids (Asn,Gln) and the occurrence of aliphatic residues are also over-represented in the many immunoglobulins whose amino acid composition around the TrpSS spatial triad closely resembles the composition around the cutinase triad (immunoglobulins part of the IGG1 triads A group). In these immonuglobulins we also observe an under-representation of the groups of charged amino acids (His, Lys, Arg)(Asp, Glu) and proline residues. Together, these parameters may be used to identify which disulfide bridges in a protein are capable of light-induced coupling to a carrier capable of coupling to thiol groups.

One such protein is goat α-lactalbumin, where the amino acid residues located within an 8 Å sphere around the tryptophan residue of the triads of goat α-lactalbumin are very similar to that of cutinase. Furthermore, it has recently been shown that upon UV excitation of goat α-lactalbumin, those disulphide bridges lying adjacent to tryptophan residues, are disrupted and free thiol groups are formed (Vanhooren A *et al*. 2002, *supra*). It is also possible to predict the UV stability of putative disulphide bridges in a given protein, solely on the basis of its primary structure, i.e., its amino acid sequence, provided that the 3D structure of homologous proteins is known and can be used for homology modelling of the 3D structure of the given protein.

It will also be apparent that known methods of recombinant DNA technology can be used to introduce amino acid substitutions into a protein sequence to create additional photo-disruptable disulfide bridges. Such substitutions may introduce a tryptophan residue or other aromatic amino acid residue in a protein as close spatial neighbour of endogenous or recombinantly engineered disulfide bridge, or alternatively a disulfide bridge may be introduced in close spatial proximity to an endogenous aromatic residue. Alternatively both aromatic and disulfide bridge may be introduced in close spatial proximity to each other. Irradiation with 295 nm light is preferable since it permits the selective excitation of tryptophan residues in a protein, which in turn may lead to the disruption of a single or a limited number of disulfide bonds. A variety of light sources suitable for the irradiation of proteins at a range of wavelengths, for the photo-induction of disulfide bond disruption include, but are not limited to, a 75-W Xenon arc lamp from a research grade spectrometer such as a RTC PTI spectrometer, a deuterium lamp, a high pressure mercury lamp. Irradiation at a single wavelength can be obtained by coupling the light source to a monochromator. A source of single and multiple photon excitation includes a high peak-power pulsed or CW laser.

New thiol groups are formed in proteins following light-induced disulfide bond disruption. Their appearance *de novo* can be measured by a 5,5'-dithiobis-(2-nitrobenzoic acid) [DTNB] based assay. In the case of cutinase, irradiated at 295nm, the formation of one thiol group per illuminated protein was detected on average. There are no free thiols in native cutinase, and disruption of the disulfide adjacent to the single tryptophan residue only yields one solvent accessible thiol that can be detected by this method. Light-induced thiol groups formed on proteins, which are accessible, will bind to any thiol binding ligand or free thiol group on a carrier.

This method of coupling to a carrier can be used to construct various types of disulfide-linked oligomers or polymers. Light-induced thiol groups in a given protein or peptide or other biomolecule can be coupled to a carrier comprising a peptide or protein. Provided that the concentration of protein and carrier molecules in the coupling reaction is sufficiently high, SS based cross-linking between neighbouring molecules will take place. While the light-induced protein should contain an SS bridge, the possession of an aromatic residue as close spacial neighbour is not essential, since the aromatic contribution to the reaction may be supplied by aromatics residues, or compounds that can mimic them, added to the coupling reaction.

This method of light-induced thiol coupling to a carrier can be usefully applied to other types of carrier molecule, such as pharmaceutical drugs, in order to facilitate their effective delivery. For example, light-induced thiol coupling of a water-soluble molecule containing a disulfide bridge (including but not limited to a peptide or protein) to a drug can help the solubilisation and delivery of water-insoluble, poorly soluble or hydrophobic drugs. Furthermore the molecule coupled to the drug may serve to protect the drug from its physiological environment, and hence improve its stability *in vivo.* This particular feature makes this technology attractive for the delivery of labile drugs such as proteins. Localized delivery of the molecule-coupled drug, by implantation at the site of treatment, would reduce systemic exposure of the patient to the drug. Carrier-linked prodrugs are generally defined as prodrugs that contain a temporary linkage of a given active substance to a transient carrier group that produces improved physicochemical or pharmacokinetic properties and that can be easily removed *in vivo,* usually by a hydrolytic cleavage. In the present invention, light-mediated disruption of the disulfide bond linking a drug to a molecule can be used to achieve a controlled release of the active drug from the molecule-coupled form, implanted in the patient. This would minimise the frequency of drug delivery to the patient, and provide for light-controlled dosing. We can also optimise the process of drug delivery, by only illuminating those regions of the body where drug release is necessary. These features would improve patient compliance, especially for drugs used for chronic indications, requiring frequent injections (such as for deficiency of certain proteins or metabolites). Controlled drug release could be induced by infra-red light (via two-photon excitation) in the case of transdermal drug delivery, within the penetration range of infra-red light, while the greater penetration of UV light (or infra-red light via three-photon excitation) would facilitate drug release deeper within the patient. Also, the use of optical fibbers allows the delivery of light at various depths in the body, as used in PDT (photodynamic therapy), as for example in the treatment of cancer/tumor patients. Since a solvent exposed disulfide bridge will be broken in a reducing environment the drug could also be released when the carrier coupled with the drug has entered a reducing environment such as the cytoplasmic space of a cell.

This method of light induced thiol coupling can also be used to immobilise a protein on a support. The most common types of bonds that are formed during coupling to a support are disulfide bonds and sulfur-metal bonds (primarily sulfur-gold) where a self-assembled layer is formed. As both types of bonds are stable, extensive washing after immobilization will not displace the protein. The density of proteins on a support can be controlled by varying the protein concentration, or the intensity and duration of UV-irradiation, and subsequently blocking the remaining activated thiol groups on the surface with reagents such as L-cysteine, (2-(2-pyridinyldithio)ethaneamine hydrochloride (PDEA) or with a thiol-lipid bilayer (Hong Q., et. al., 2001, Biochemical Society Transactions 29(4):587-582). The support, with evenly distributed immobilized proteins, is therefore blocked to prevent non-specific binding. According to the present invention the method of immobilization does not involve any chemical steps, since the thiol-activated proteins formed by UV radiation, spontaneously self-assemble on the support. The described thiol and disulfide exchange reactions are an effective and rapid way to bind molecules to supports. A particular advantage of the present invention is its avoidance of the several disadvantages associated with the chemical generation of free thiols in a protein. Some proteins, e.g., cutinase, are inactivated by the reducing agents (DTT or beta-mercaptoethanol) used to generate free thiols, as shown in Example 10. If a reducing agent comprising a thiol group, is used to chemically generate free thiols in a protein, it must be removed before immobilisation by can be performed, during which step the disulfide bonds can reform. Alternative reducing agents, such as 2-carboxyethyl)phosphine, lacking a thiol group, have the disadvantage that they are reactive to other groups. The use of reducing agents to disrupt disulfide bonds has the additional disadvantage that they are pH dependent, both with regards their chemical stability and their reducing activity.

Immobilisation of a protein on a support can also be spatially controlled. Present day laser technology allow for focal spots with dimensions of 1 micrometer or less. If a specific protein or target molecule, containing SS bridge(s), is incubated with a thiol-binding support, light-induced thiol group formation and coupling could be limited to the focal points of illumination. The viscosity of the solution should be controlled to minimise diffusive processes that might disperse the illuminated molecules beyond the spot size. This approach would allow for an extremely dense packing of identifiable and different molecules on a support surface. Thus the method of the present invention could be used for charging microarrays with molecules.

In a further aspect of the present invention, the orientation of the immobilized protein can be controlled in a uniform and reproducible manner. Prolonged selective excitation of tryptophan residues in a protein will only lead to disruption of those disulfide bridges to which excitation energy is transferred. The location of these photo-disruptable disulfide bridges, forming free thiol groups, can be predicted from the protein's structure, where it is known from three-dimensional models, nuclear magnetic resonance (NMR) or X-ray diffraction crystallography analysis. In those cases where only a single thiol group is induced by irradiation of tryptophan residues in a protein, as is the case for cutinase, then immobilisation of the protein on a support will occur exclusively via this thiol group. In contrast to alternative methods of disulfide bridge disruption and thiol-group formation, such as the use of reducing agents, the light-induced method of the present invention leads to targeted disruption of disulfide bridges forming one or only a few accessible thiol groups, whose position can be precisely predicted. The subsequently immobilised proteins will thus have a single or very limited number of orientations. Since cutinase has only a single thiol group for immobilisation, which is distant from the active site, the accessibility of substrates will not be limited by immobilisation. Immobilisation via a surface accessible thiol group, remote from the protein's active site, as is the case for cutinase and lysosyme, is also less likely to alter the conformation or structural properties of the protein. In other words, the immobilisation method of the present invention serves to preserve the native state of the immobilised protein. All functional/structural assays performed on proteins, which are immobilised in a uniform orientation according to the methods of the present invention, will generate data derived from a uniform population of proteins. The structural and functional uniformity of the immobilised proteins, and retention of their native state, is of primary importance for screening or assaying proteins for catalytic, binding, or any other biological properties and provides one of the many valuable advantages of the present invention. In the case of proteins such as lysosyme, which have anti-microbial properties, it is useful to be able to immobilise said proteins on a surface (e.g. food, skin, packaging) in order to prevent microbial growth and infection.

## Examples

### Example 1: Formation of free thiol groups in cutinase upon UV illumination

The disruption of disulfide bridges in a protein following UV illumination was examined using a cutinase, with lipase/esterase properties isolated from *Fusarium solani pisi.*

*Steady-sate fluorescence emission intensity of cutinase*

Cutinase preparations were subjected to UV irradiation at 295 nm under the following conditions in order to follow its steady-state fluorescence emission intensity of continuous illumination: Three ml of 2μM stock solution of cutinase was continuously illuminated at 295 nm for increasing periods of time (0h, 1 h, 2h, 3h, 4h, and 5h) in a quartz macro-cuvette (1 cm path length). Light excitation at 295 nm was supplied by a Xenon Arc Lamp coupled to a monochromator provided by a RTC 2000 PTI spectrometer. The cuvette was mounted in a thermostated cuvette, kept at a constant temperature of 25˚C. The cutinase sample was maintained as a homogeneous solution by continuous stirring at 700 rpm with a magnet. Excitation and emission slits were set at 6 nm. The fluorescence intensity of cutinase at 350 nm was continuously monitored throughout 5h of illumination. The time dependent fluorescence emission intensity of a 2μM cutinase solution at 350 nm upon illumination with 295nm light is shown in Figure 1. Fluorescence intensity increased very rapidly over the first 7200s, followed by a plateau where the fluorescence yield appeared to stabilise.

*Detection of free thiols in cutinase following UV illumination*

The concentration of free thiol groups in cutinase formed following UV illumination was determined as follows: Thiol groups on the cutinase were detected and quantified by a spectrophotometric assay based on the reaction of thiol groups with 5,5'-dithiobis-(2-nitrobenzoic acid) [DTNB], as shown in figure 2, or Ellman's reagent (Ellman GG,. 1959 Arch. Biochem. Biophys. 82: 70-77; Hu ML., 1994, Meth. Enzymology 233: 380-385). Three ml of a 17.3μM solution of cutinase in 20mM TrisHCl pH 8.5 was illuminated with 295nm light in a quartz macro-cuvette (1 cm path length) for different periods of time using a RTC 2000 PTI spectrometer, as described above. The time dependent fluorescence emission intensity of the sample at 350nm was measured. All slits were set to 2 nm band-width. An excess of DTNB (100μl of an 8.5mM DTNB stock solution in absolute methanol) was added to 900μl of cutinase solution, prior to or after its illumination at 295 nm. The stock solution of DTNB in methanol is stable for up to 2 weeks at 4˚C (Hu ML., 1994 *supra*). Immediately after mixing the two components, absorbance of the released NTB ion (nitrothiobenzoate ion, $\varepsilon_{412nm}=13600M^{-1}cm^{-1}$) was measured at 412 nm with a UV/Visible Pharmacia spectrophotometer, and again after 20 and 24 min reaction time at 25˚C. The free thiol concentration is proportional to the absorbance value at 412 nm. The readings were stable between 20 and 24 min. Each data point was an average of three measurements after 24 min. The control sample comprised 100μl of a 8.5mM DTNB stock solution in absolute methanol mixed with 900μl of non-irradiated cutinase (17.3μM cutinase solution in 20mM TrisHCl pH 8.5). The concentration of illuminated cutinase sample was raised to ensure that the amount of free thiol groups formed upon illumination were above the detection limit of the DTNB method.

Free thiol groups were not detected in non-illuminated control samples of cutinase using the DTNB assay. This is consistent with the absence of free thiol groups in native cutinase, since all four cysteine residues in the enzyme are involved in disulphide bridges. The amount of thiol groups formed in the cutinase sample during illumination was measured as a function of the fluorescence intensity increase (F/Fo). Figure 3 demonstrates a positive correlation between the time-dependent increase in Trp fluorescence emission intensity at 295 nm of cutinase upon UV excitation, and the increased concentration of free thiol groups detected at different ratios of fluorescence emission increase (F/Fo).

These results support the working model that the initial fluorescence intensity of tryptophan (Trp) is highly quenched due to the presence of a nearby intact disulfide bridge, and that the fluorescence intensity increase of the single endogenous Trp in cutinase is due to the cleavage of the nearby disulfide bridge upon illumination of cutinase at 295 nm.

**Example 2: Irradiation-induced disruption of a disulfide bridge in native cutinase is dependent on the presence of a Trp residue as spatial neighbour**

In order to demonstrate the role of a tryptophan residue in cutinase in the irradiation induced disruption of a disulfide bridge, the Trp fluorescence emission intensity of the native protein was compared to that of reduced cutinase in which all disulfide bridges were chemically disrupted. The cutinase protein was partially denatured by heat in order to facilitate reduction of all its disulfide bridges. The Trp fluorescence emission intensity of the native and denatured cutinase, following irradiation, was used to demonstrate the importance of a Trp residue being a spatial neighbour of a disulfide bridge, for the transfer of excitation energy from Trp to the disulfide bridge.

*Reduction of native cutinase with DTT*

In native cutinase, the disulfide bridges within the folded protein are inaccessible to solvent, and they cannot be directly reduced by DTT. However, if the cutinase is heated in a pH 8.5 buffer to a temperature exceeding its unfolding temperature, the subsequent unfolding process will facilitate the access of DTT to the protein's disulphide bridges. The heat denaturation step was performed on a dilute solution of cutinase, in order to avoid precipitation of the protein. The buffer (20mM Tris-HCl 8.5), selected for the heat denaturation of cutinase, displays a minimal pH drift in relation to temperature and volume changes, and furthermore Tris-HCl has minimal enthalpy of ionisation. The concentration of cutinase solutions was estimated from the OD 280nm of the solution, using the extinction coefficient of cutinase at 280nm (13500 $M^{-1}cm^{-1}$).

Reduction of cutinase disulfide bonds with DTT was performed as follows: A 650μl sample of 1μM cutinase suspended in 20mM TrisHCl pH 8.5 was heated from 25°C to 70°C, to which excess DTT was added (8.5 μl of 3.7M DTT in 20mM TrisHCl pH 8.5), to give a final concentration of 50mM DTT.

*Trp fluorescence emission intensity measurements of native or reduced cutinase*

Emission spectra of 1μM cutinase samples, upon excitation at 295 nm, were measured under the following conditions with a RTC 2000 PTI spectrometer, with slits set at 2 nm band width: (A) cutinase incubated at 25°C without DTT; (B) cutinase incubated at 70°C without DTT; (C) cutinase incubated at 25°C without DTT, following heating to 70°C and cooling to 25°C; (D) cutinase incubated at 25°C with DTT, following heating to 70°C, addition of DTT and cooling to 25°C. The spectra were corrected for Raman contribution.

The four emission spectra are shown in Figure 4. The emission spectrum of cutinase incubated at 25°C (A) is the same as the emission spectrum of cutinase after cooling from 70°C to 25°C (C), demonstrating that thermal unfolding of cutinase at pH 8.5 is a reversible process. This can be compared with the emission spectrum of cutinase in an unfolded state (B). The Trp emission fluorescence intensity of cutinase at 25°C, after heating to 70°C in the presence of DTT (D) was greater than for native cutinase without DTT, as in (A). These spectra serve to confirm the correlation between an increase in Trp fluorescence emission intensity of cutinase (upon excitation at 295nm) and reduction of its disulfide bridges, shown in Example 1.

In the course of obtaining successive spectra for samples (A) as well as (C), upon continuous excitation of the Trp residue of cutinase at 295nm, the fluorescence emission was observed to increase, as shown in Example 1. The similar fluorescence increase of samples (A) and (C) is consistent with the close proximity between the Trp and the disulphide bridge observed in the 3D structure of native cutinase upon refolding. This increase in fluorescence emission, upon continuous excitation of the Trp residue of cutinase at 295 nm, was not observed for samples (B) or (D). These data support the conclusion that a close proximity between the Trp residue and the disulphide bridge in cutinase is necessary for the photo-induced mechanism, and that this proximity is lost when the protein is unfolded at 70°C, pH 8.5. This is the most likely reason why the Trp fluorescence quantum yield of cutinase at 70°C was higher than at 25°C, despite the fact that one would expect a lower quantum yield due to the higher temperature causing collisional quenching between the Trp and solvent molecules.

**Example 3. Specificity of light-induced disulphide bridge disruption in proteins**

The cutinase gene of *Fusarium solani pisi* has been mutated to encode a mutant cutinase polypeptide where the single tryptophan residue is replaced by alanine, a non-fluorescent amino acid (W69A). The absence of light-inducible disulfide bridge disruption in the mutant in comparison to native cutinase, demonstrated below, further confirms the requirement for an aromatic amino acid (e.g. trp) in close spatial proximity to the disulfide bridge. The mutant cutinase (W69A) was expressed recombinantly. Three ml of a 2 μM solution of the mutant or native protein, in 50 mM Tris-HCl pH 7.0, was transferred to a cuvette, thermostated at 25° C, with magnetic stirring at 700 rpm, and illuminated at 296 nm using a 75W xenon lamp from an RTC 2000 PTI spectrophotomoter coupled to a monochromator, with an excitation and emission slit of 10 and 2 nm, respectively. As can be seen from the absorption (A) and emission spectra (F) of the aromatic amino acids in aqueous solution at pH7, shown in Figure 5, tryptophan is the only aromatic residue to be excited by light at a wavelength of 296 nm. The mutant or native cutinase solution (1ml), before or after illumination at 296nm, was incubated with the intramolecularly quenched probe BODIPY FL L-cystine (Figure 6), at a final concentration of 20 μM, for 20 min in the dark, at 25°C. Each cutinase sample, comprising BODIPY FL L-cystine, was then excited at 480nm (excitation wavelength of BODIPY FL L-cystine) and the emission spectrum recorded between 500 and 620nm, using excitation and emission slits set at 4 and 2nm, respectively. Any thiol groups, generated in the cutinase sample during illumination, will react with the SS group of the probe, Bodipy FL L-cystine (that contains two bodipy groups), leading to the breakage of this molecule and consequent increased distance between the two bodipy groups. This in turn leads to an increase of the fluorescence emission intensity of each bodipy group. As seen in Figure 7, illumination of mutant cutinase (A), in contrast to the native cutinase (B), does not give increased Bodipy fluorescence, indicating the absence of thiol group formation in the mutant. This demonstrates that, upon 296 nm illumination, the disulfide bridge in the mutant cutinase adjacent to the W69A mutation remains intact, due to the absence of a Trp residue in close spatial proximity.

The identity of the disrupted disulfide bridge in native cutinase following UV illumination was confirmed by further studies. Native cutinase is known to have two disulfide bridges, one of which is located close to the active site and its integrity is essential for catalytic activity, and asecond which is remote from the active site, and close to a Trp residue. Since the native cutinase was found to retain its enzymatic activity following UV illumination, it is evident that only the surface localised disulfide bridge is disrupted. NMR and Mass spectrometry data by Prompers et al., 1999, FEBS Lett. 456: 409-16. also shows that the SS bridge disrupted by UV illumination is the one close to the Trp residue. TThe formation of DTNB detectable thiol groups in native cutinase following UV irradiation provides an additional tool for quantitating the disruption of disulfide bridges. Following UV illumination only one thiol group per cutinase molecule was detected. This is consistent with the disruption of the surface localised disulfide bond, where the second thiol group is not solvent accessible. At least one of the cystine residues of the second disulfide bridge is solvent accessible, but disruption was not detected.

**Example 4. Prediction of proteins with light-inducible Trp-SS triads**

Proteins comprising disulfide bridge(s) and aromatic residue(s), wherein a carbon or nitrogen atom in the indole ring of a selected (trp) residue lies within, or less than, 5 Å from the S atoms of a disulfide bridge were identified in the Protein Data Bank of the Research Collaboration for Structural Bioinformatics (RCSB) (Bergman et al. 2000, Nucleic Acids Research, 28: 235-242). Only those proteins for which either NMR or X-ray crystal structures were available were considered. The distances between the atoms of the triad were measured using the molecular visualization program: Deep View/Swiss-Pdb Viewer at www.expasy.org.spdbv (Guex, N and Peitsch, M.C., 1997, Electrophoresis 18: 2714-2723). The selected proteins were matched against a sorted list of proteins having less than a 90% sequence identity and belonging to each of the different enzyme classes [EC Number and classification]. For the purposes of this analysis those proteins (hydrolases) in which the trp-SS triad was located in a ligand were not considered. Furthermore, triads occurring more than once in a PDB entry due to molecule dimers, trimers, etcetera or repetitions of the molecule in the unit cell were only considered once.

Trp-SS triads were found to be common among hydrolase enzymes, of which cutinase is a member. The amino acid residues located within an 8Å, 12Å and 15Å radius of the Trp-SS triad(s) identified in each of these proteins (where the centre of the spherical area is the indole ring of the tryptophan located closest to the disulfide bond) was calculated and grouped according to their properties: Gly, Ala, Val (short aliphatic); Leu, Ile (long aliphatic); Pro; Ser,Thr (hydroxyl-containing); Cys, Met (sulfur-containing); Asn, Gln (amidic); Asp, Glu (acidic); His, Lys, Arg (basic); Phe, Tyr, Trp (aromatic). The average frequency of occurrence of each group of amino acids in proteins in general, has been determined by Mathews, Christopher K. & van Holde, K. E., in "Biochemistry" 2nd Edition, The Benjamin/Cummings Publishing Company, Inc., 1996, ISBN: 0-8053-3931-0, and are 23.4% (Gly, Ala, Val); 12.1% (Leu, Ile); 4.6% (Pro); 13.1 (Ser, Thr); 4.5 (Cys, Met); 8.3% (Asn, Gln); 11.7% (Asp, Glu); 13.8% (His, Lys, Arg) and 8.1% (Phe, Tyr, Trp).

The occurrence of each residue was counted and ordered in groups according to the properties of the residues. The contribution from the triad residues was removed by subtracting 1 tryptophan from the number of aromatic residues and subtracting 2 cysteines from the number of occurrences in the sulphur containing residues from each sphere residue list. Then the frequency of occurrence in the subsets, f(subset), for every group was calculated as

Eq. 2.1 f(subset) = no. of residues in group / no. of residues in total in subset.

The f(subset) was used to calculate a fractional score as being the frequency of occurrence in the subset divided by the frequency of occurrence in proteins in general (see table 2.1):

Eq. 2.2 Score = f(subset) / f(protein)

Therefore: "scores" > 1 means that residue/group is overrepresented "scores" < 1 means that residue/group is under-represented

Having the score for each group of residues arranged in columns in the spreadsheet for each triad the similarity of the triads with the one of cutinase (W69, S31, S109) was calculated using the Correlation tool of the Data Analysis Tool Pack. This operation yielded a correlation coefficient as a measure of the similarity between the triad of cutinase and triad(n). The correlation coefficients, , are calculated as:

Eq. 2.3. $$\rho_{t[cut],t[n]} = \frac{\operatorname{cov}ariance(t[cut],t[n])}{\sigma_{t[cut]} \cdot \sigma_{t[n]}}$$

where t[cut] and t[n] denotes the triad of cutinase and the triad n respectively, and is the standard deviation of the triad in consideration.

The correlation coefficients were used to sort the triads with respect to their similarity to cutinase. The graphical representation of the result was made as a contour plot of the scores with the scores less than one representing groups of residues occurring less than expected and scores of one and higher representing groups of residues occurring at expected and more than expected frequencies.

Particularly within an 8Å radius, a specific subset of amino acid residues were identified, which were clearly distinct from the average frequency of occurrence of amino acids in a protein in general, particularly with regard the abundance of the residues (Phe, Tyr, Trp) (Asp, Glu) (Asn, Gln) and Pro (Figure 9. Thus, the amidic residues (Asn, Gln) are over-represented, and in the majority of cases the occurrence of short aliphatic residues (Gly, Ala, Val) and/or long aliphatic residues (Leu, Ile) are also over-represented. In almost every triad there is an under-representation of the groups of charged amino acids (His, Lys, Arg)(Asp, Glu) and proline residues.

The conservation of a specific subset of amino acids, seen within an 8Å radius of the triad, was less apparent at 12Å and 15Å. Analysis of the amino acid residues located within an 8Å radius proximity of trp-SS triads in immunoglobulins, revealed an over-representation of hydroxyl-containing amino acids, and in many IgG Trp S-S triads the aromatic residues are also over-represented. One or both amidic amino acids (Asn,Gln) and the occurrence of aliphatic residues are also over-represented in the many immunoglobulins whose amino acid composition around the TrpSS spatial triad closely resembles the composition around the cutinase triad (immunoglobulins part of the IGG1 triads A group). In these immonuglobulins we also observe an under-representation of the groups of charged amino acids (His, Lys, Arg)(Asp, Glu) and proline residues (Figures 11 and 12). Those Trp-SS triads in which the angle between the plane of the emitting dipole of the aromatic Trp side chain is orthogonal to the plane of the absorbing dipole of the disulfide bridge are unlikely to exchange excitation energy between excited state Trp and a disulfide bridge.

The light-inducible properties of some members of this group of proteins, which comprised both a Trp-SS triad and a subset of amino acid residues, similar to that found within an 8Å radius of the Trp-SS triad of cutinase, were examined. Each protein was assayed for light-inducible disulfide bridge disruption using the intramolecularly quenched probe BODIPY FL L-cystine (Figure 6) as described in Example 3 for the mutant cutinase. The following proteins: hen egg white lysosyme, *Rhizopus niveus* triglyceride lipase, human plasminogen, human placental alkaline phosphatase, chymosin B, and human immunoglobulin were all shown to comprise light-inducible Trp-SS triads, as shown in Figure 8 A-F.

The protein alpa-lactalubumin comprises 4 disulfide bridges, of which only two are disrupted on UV illumination, namely Cys6-Cys120 and Cys73-Cys91 (Vanhooren et al (2002) Biochemistry, 41 (36):11035-11043). Although both Cys73-Cys91 and Cys28-Cys11 in alpha-lactalbumin have at least one sulphur atom located within 4Å of the nearest atom of a tryptophan indole ring, only the former is susceptible to light-induced disruption. In contrast to the triad Trp118-Cys28-Cys111, the triad Trp60-Cys73-Cys9 is located within an 8Å radius spherical area whose amino acid composition is similar to that of the cutinase triad, particularly with regards the abundance of the (Phe, Tyr, Trp), (Asp, Glu), (Asn, Gln) and Pro residues (Figure 10).

**Example 5. Light induced cutinase coupling.**

The formation of free SH groups in the model protein cutinase from *Fusarium solani pisi*, as well as several other proteins, in response to illumination has been shown in Examples 1, 2 and 3. The reactive thiol group of the cutinase can then be coupled to carrier molecules in solution that have either a free and solvent-accessible thiol group or a solvent accessible disulfide bridge. The method of light induced cutinase coupling to carrier molecules in solution is illustrated by the coupling of a free thiol group in cutinase, induced by irradiation with light of 295nm, to a disulfide group of DTNB and BODIPY FL L-cystine, as shown in figures 2 and 6. Light induced coupling between BODIPY FL L-cystine and each of the proteins lysosyme, lipase, plasminogen, alkaline phosphatase, chymosin, immunoglobulin are also demonstrated in example 3 (Figure 8).

The coupling reaction was performed as follows. Three ml of a 17.3μM solution of cutinase in 20mM TrisHCl pH 8.5 was illuminated with 295nm light in a quartz macro-cuvette (1 cm path length) for different periods of time using a RTC 2000 PTI spectrometer, as described in example 1 and 2. An excess of DTNB (100μl of an 8.5mM DTNB stock solution in absolute methanol) was added to 900μl of cutinase solution, prior to or after its illumination at 295 nm. Immediately after mixing the two components, absorbance of the released NTB ion (nitrothiobenzoate ion,

$\varepsilon_{412nm}$=13600$M^{-1}cm^{-1}$) was measured at 412 nm with a UV/Visible Pharmacia spectrophotometer, and again after 20 and 24 min reaction time at 25˚C. The coupling reaction with DTNB results in the formation of a stoichiometric amount the NTB ion. Thus, the concentration of coupled cutinase is proportional to the absorbance value at 412 nm. The control sample comprised 100μl of an 8.5mM DTNB stock solution in absolute methanol mixed with 900μl of non-irradiated cutinase (17.3μM cutinase solution in 20mM Tris-HCl pH 8.5).

**Example 6. Light induced protein immobilisation.**

The free reactive thiol groups formed in a protein e.g. cutinase from *Fusarium solani pisi*, in response to illumination (as shown in Examples 1, 2 and 3), are used to link the protein to a thiol reactive support or carrier molecule, whether it be gold, or gold derivatised with thiol groups, or quartz surfaces derivatised with SH groups, or a polymer support or carrier molecule derivatised with SH groups. The immobilised proteins are shown to retain their functional properties (e.g. enzymatic).

A polymer support is derivatized with SH groups by first activating with NHS/EDC which modifies carboxymethyl groups to N-hydroxysuccinimide esters. A thiol group is then introduced on the support by incubation with cysteamine in 0.1 M borate buffer pH 8.0 and subsequently with DTT or DTE (dithioerythritol) in 0.1M borate buffer pH 8.0. The support is flushed with 0.1 M borate buffer pH 8.0 prior to immobilization.

Proteins may also be linked to a quartz support, derivatised with SH groups, and the immobilisation monitored by Total Internal Refection Fluorescence (TIRF) spectroscopy, as exemplified for an number of proteins, using the following procedure:

Step 1: Surface preparation of quartz slides for TIRF spectroscopy

The quartz slide (12 $cm^2$) was cleaned by immersion in chromosulfuric acid (Merck 1.02499/Z624399) for 1 hour at 70-75 ˚C, and then rinsed in water at ambient temperature. The slide was then hydroxylated by immersion in 5 w/v% potassium persulfate (99% $K_2S_2O_8$, Acros Organics 20201-000) in deionised $H_2O$ for 1 hour at 99-100˚ C, in order to increase the number of OH groups. The hydroxylated slides were rinsed with deionised $H_2O$ and dried rapidly.

SH-activated slides are then prepared by applying 150μl 0.03% v/v 3-mercaptopropyl-trimethoxysilane (Merck 63800) in m-xylene (99+%, Acros Organics 1808600100) to the hydroxylated slide. The xylene solvent was allowed to evaporate completely from the slide, and the surface was then flushed successively with pure xylene; ethanol and deionised $H_2O$, and finally dried to yield a uniform, optically perfect silane coating.

Step 2: Protein immobilisation on quartz slides by TIRF spectroscopy

The SH-activated, or control hydroxylated, slide was mounted on the quartz prism with glycerol, and attached to the flow chamber with a 10 μm polyurethane gasket, and assembled in the TIRF instrument coupled to a fluorescence spectrophotometer, supplied with a 75W xenon arc source. Protein immobilisation was performed by flushing the flow chamber at a flow rate of 0.25 $ml^{-1}$, or incubating with the following solutions:

a) 25 mM Tris-HCL, pH 8.5 (buffer A), for 5-10 min, to hydrate the slide.
b) 0.5-2.0 μM protein solution in buffer A at 25˚C for 10 - 20 min.
c) Buffer A for 10 min.
d) Non-ionic detergent e.g. 2 v/v% Helmanex II (Hellma GmbH & Co KH, Germany) for 5 min.
e) Buffer A

Protein immobilisation was performed under conditions of continuous UV illumination, limited UV illumination (10 min light, or 1 sec light/min) at 280 or 296 nm, or darkness (control) and was monitored by fluorescence emission at 330nm. The slides were rinsed with ethanol and $H_2O$ before measuring immobilised protein activity.

In a modification of the above procedure, the protein sample, e.g., 2 μM cutinase solution, is UV-irradiated and subsequently immobilised on the SH-derivatised quartz slide by incubation and/or flushing with the sample, followed by purging with buffer A, and optionally a detergent to remove unbound protein.

Step 3: Enzymatic activity of immobilised activity

A fluorogenic enzyme substrate was applied to the quartz slide following the immobilisation procedure, and the reaction products were detected by fluorescence spectroscopy.

A. UV-induced immobilisation of cutinase

Immobilisation of cutinase on SH-activated and hydroxylated quartz slides under continuous or limited illumination was monitored by detecting fluorescence at 330nm, as shown in Figure 13. The flow chamber was flushed (A-B), and then incubated (B-C) with 2.0 μM cutinase solution; rinsed with buffer A (C-D), and rinsed with detergent and buffer A (E). Cutinase that was not tightly bound to the quartz slide was removed during flushing with buffer and detergent. The relative efficiency of cutinase immobilisation, deduced from fluorescence emission intensity, was greater under continuous illumination than limited illumination, and was dependent on an SH-activated quartz surface (Figure 14A) and on illumination (Figure 14B). Immobilisation under limited light conditions can be used to reduce the risk of protein photobleaching. Mutant cutinase (W69A), which does not exhibit light-inducible disulfide bridge disruption, was not immobilised on SH-activated quartz slides under the same conditions (Figure 14B).

The enzymatic activity of cutinase, immobilised on quartz slides, was detected by measuring ester bond cleavage of the non-fluorescent substrate 4-methylumbelliferyl butyrate, with the release of fluorescent 4-methylumbelliferone, according to the following procedure:

a) 25-100μl of 10μM 4-methylumbelliferyl butyrate substrate in 25mM Tris-HCl, pH 8.0 was deposited on the surface of the quartz slide.
b) Incubating the slide at room temperature for 30 minutes.
c) Fluorescence emission intensity (450nm) of the slide, upon UV excitation (365nm), was monitored by digital camera. (Alternatively the reaction mixture can be transferred to a microtiter plate well and fluorescence emission monitored by UV/VIS spectrophotometer.)

The substrate, 4-methylum-belliferylbutyrate, is dissolved in DMSO, yielding a 25.2 mM stock solution, which is added to the assay buffer.

Active cutinase enzyme was detected on a SH-activated quartz slide but not a hydroxylated quartz slide (Figure 15), which further confirms the observation that light-induced coupling of cutinase to the SH-activated quartz support is via a thiol group.

B. UV-induced immobilisation of lysozyme and chymosin

Immobilisation of a 2μM lysozyme or chymosin solution on quartz slides was performed and monitored by TIRF spectroscopy as described above for cutinase. Lysozyme immobilisation was shown to be light-activated and to be dependent on SH-activation of the quartz slide (Figure 16A versus B). The coupling efficiency of lysosyme immobilisation was found to be enhanced if the continuous illumination was limited to 10 min. Chymosin immobilisation was similarly found to be light activated and dependent on coupling to an SH-quartz surface (Figure 16 C).

Detection of lysosyme actity: lysozyme activity immobilised on a quartz slide was detection according to the procdure used for the detection of cutinase activity, with the exception that a lysozyme-specific substrate was used (EnzCheck Lysozyme, 22013, Molecular probes Inc), and that the fluorescence signal was recorded spectrometrically. A drop of 125 μL of 50μg/ml substrate (in 0.1M Phosphate / 0.1M NaCl pH 7.5) was applied on the quartz slide. After 30 minutes incubation of at ambient room temperature (20-25˚C), 100 μL of the solution was transferred to a well in a black multiplate (96 wells, Nunc 267742). In a fluorescence multiplate reader (SpectraMax Gemini XS) the samples were excited at 495 nm and the fluorescence emission intensity (FEI) at 525 nm was recorded. The concentrations and procedures were based on the protocol which follows the EnzCheck Lysozyme activity assay kit 22013 from Molecular probes Inc.

Lysozyme activity analysis also showed show larger activity on SH-activated slides with UV-illumination compared to the controls, comprising immobilisation/adsorption on SH-activated slides in dark, or on hydroxylated slides with UV.

Detection of chymosin activity is performed with EnzCheck Protease kit E6639 (Molecular Probes Inc.) using a substrate concentration of 10μg/mL in 10 mM Tris pH 7.8 (buffer contained in kit). A 125 μL drop of substrate was applied on the quartz slide. After an incubation time of 30 minutes at ambient room temperature (20-25˚C), 100 μL of the solution was transferred to a well in a black multiplate (96 wells, Nunc 267742). In the fluorescence multiplate reader (SpectraMax Gemini XS) the samples were excited at 589 nm and the fluorescence emission intensity (FEI) at 625 nm is recorded. The concentrations and procedures are based on the protocol which follows the EnzCheck Protease activity assay kit E6639 from Molecular probes Inc.

C. UV-induced immobilisation of immunoglobulin

Proteolytic digestion of immunoglobulin IgG complexes with papain generates two F(ab) fragments, each comprising two polypeptides. The available 3D structures of domains of Fab fragments of IgG immunoglobulins shows that all of them are rich in Trp/Cys-Cys triads. The available 3D structures of Fab fragments show that the intra-domain disulphide bridges present in the $C_H$, $V_H$, $C_L$, anc $V_L$ domains are not solvent accessible. An additional inter-domain disulphide bridge is present connecting conserved regions at the base of the Fab fragment, located far from the antigen biding site. This disulphide bridge is solvent accessible and located nearby a Trp residue.

Orientated immobilisation of F(ab) fragments on an SH-group derivatised support can be achieved by UV-induced disruption of a disulfide bridge of the F(ab) fragment, e.g. the C-terminal interdomain disulfide bridge (Cys136-Cys214) of 1CBV mouse IgG1 F(ab), which lies within 10-11Å of Trp196. The antigene binding site, located at the other end of the Fab fragment, will be remote from the site of immobilisation and thus maintain its availability to antigene recognition.

The following procedure illustrates the purification of F(ab) fragments, their UV-induced immobilisation, and detection of functionally active F(ab) fragments coupled to a support:

a) Mouse IgG1 (Sigma M7894) is digested with papain (Sigma P4762) according to the procedure of Aybay et al 2003, Immunology Letters 85: 231-235, and F(ab) fragments may be purified using the ImmunoPure F(ab) kit supplied by Pierce Biotechnology, Illinois, USA.

b) A 1-10 μM F(ab) fragment solution in 25 mM Tris-HCl, pH 8.0, is immobilised on an SH-activated quartz slide in the flow cell of a TIRF instrument, as described above, with the aid of UV illumination of wavelength from 280nm to 296nm. Aromatic amino acids can be included in the immobilisation buffer to facilitate light-induced disruption of inter- or intra-domain disulfide bridges of a protein that are not in proximity of an endogenous aromatic amino acid, which can also be usefully employed to the immobilisation of immunoglobulin F(ab) fragments. The specificity of coupling is tested by performing control immobilisation assays in the dark and on a hydroxylated support. Fluorescence emission at 330nm on excitation at 296nm, provides a measure of F(ab) immobilised on the slide.

c) The antigen binding activity of F(ab) fragments, immobilised in step (b), is determined by introducing an anti-mouse IgG F(ab) specific fluorescein isothiocyanate conjugate (Sigma F2653) diluted 100x in Tris-HCl pH 8.0, into the flow cell. Following 10-30 min incubation at 25° C, unbound conjugate is removed by flushing the flow cell with buffer A, and the fluorescence emission intensity of the conjugate bound to the quartz slide is then detected at 525nm, on excitation at 495nm.

D. UV-induced immobilisation of glucose oxidase

Glucose oxidase is used to quantitate β-D-glucose in liquids (e.g. human blood). Glucose oxidase comprises a solvent accessible trp-SS triad (Cys164-Cys206) located 7-9Å from Trp133, and light-induced disruption of this disulfide bridge creates a thiol group suitable for coupling to a carrier. Since the disulfide bridge is positioned remote from the active site, its disruption, and the subsequent coupling of the generated thiol to a support , will not impair the catalytic activity of glucose oxidase. Immobilisation of a 2μM glucose oxidase solution on quartz slides is performed and monitored by TIRF spectroscopy as described above for cutinase.

**Example 7. Spatially controlled protein immobilisation**

Cutinase molecules were immobilised on SH-activated quartz slides, spatially confined to an area of 1-2 $\mu m^2$. A droplet of a 5μM cutinase solution in 25mM Tris-HCl pH 8.5 was deposited on the SH-activated quartz slide at room temperature. The droplet was illuminated with laser light of 296nm, whereby the tip of the laser probe was submerged in the droplet and positioned 1-2mm above the activated quartz surface. Immobilised cutinase activity was detected on the slide surface, in a spatially confined area, using the cutinase assay given in Example 6 A.

**Example 8. Reversible protein immobilization with UV light**

UV light is used to release a protein e.g. a cutinase enzyme, disulfide bonded to a thiol reactive surface by light-induced immobilisation. When tryptophan residues and disulfide bridges are present in solution, the irradiation of the tryptophan disrupts the disulfide bridge by a photo-induced mechanism. The immobilised cutinase is released by UV light irradiation according to the following steps:

1. Placing the slide with immobilized cutinase in a TIRF apparatus and monitoring the adsorbed protein concentration on the slide by fluorescence emission at 350nm.
2. Purging the slide with a continuous flow of 20mM Tris-HCl pH8.5 buffer containing 20 μM tryptophan, or an aromatic amino aicd residue or a compound that mimics an aromatic residue.
3. Irradiating the slide with 295nm light for 5 hours.

4. Purging the slide with 20mM Tris-HCl pH8.5 buffer.
5. Measuring fluorescence of the slide at 350nm continuously, to follow protein elution.

**Example 9. Reversible immobilization with reducing agent**

The cutinase enzyme, disulfide bonded to a thiol reactive surface by light-induced immobilisation, is released by chemical reduction of disulfide bonds. Thereafter, the protein is dialysed in a 20mM Tris-HCl pH8.5 buffer, free of reducing agents, to regain its native structure. The immobilised cutinase is released by a reducing agent according to the following steps:

1. Placing the slide with immobilized cutinase in a TIRF apparatus and monitoring the adsorbed protein concentration on the slide by fluorescence at 350nm.
2. Purging the slide with a continuous flow of buffer (20mM Tris-HCl pH8.5 containing 50mM DTT) for 30 min, 1 h or up to 5 hours.
3. Purging the slide with 20mM Tris-HCl pH8.5 buffer.
4. Measuring fluorescence of the slide at 350nm continuously, to follow protein elution.

**Example 10: Effect of light irradiation or a reducing agent of protein** function and stability

A. Effect of light-irradiation on cutinase catalytic activity

A protein sample (cutinase) illuminated at 296 nm showed no decrease in activity compared to a protein sample placed in darkness or in normal (artificial) laboratory light - at least up to approx. 3 hours of illumination (Figure 17 A).

Cutinase (1 μM in 25 mM Tris pH 8.5) was illuminated in a PTI fluorescence spectrometer (excitation slit was set at 6nm and emission slit were set at ½ nm). 3ml sample was placed in a quartz cuvette and was continuously stirred (500rpm, 7x2mm magnet stick) during the continuous illumination at 296 nm (emission was recorded at 330 nm). Throughout the illumination period 5μL samples were collected for immediate activity measurement (within 5-10 minutes). A control sample was placed on the laboratory bench under artificial light source (neon lamp), and as reference a sample was placed in darkness.

Activity of all three samples were measured (in duplicate). For control purposes, the pure substrate in buffer was analysed as well. 100 μL substrate solution (10 μM 4-methylum-belliferylbutyrate, Fluka 19362, in 25mM Tris pH 8.0) was pipetted into 8 wells in a black multiplate (96 wells, Nunc 267742). In a fluorescence multiplate reader (SpectraMax Gemini XS) the samples were excited at 365 nm and the fluorescence emission intensity (FI) of the product at 450 nm was recorded every second minute during the initial 8-10 minutes. The temporal increase in fluorescence (delta FI per minute) was used as a measure of protein activity (during the initial 10min the Fluorescence emission intensity increased linearly with.the reaction time, $r^2$>0.98). The reported activity is the ratio between the activity of the sample under UV illumination or the control sample in artificial light divided by the activity of the reference sample placed in dark. Activity was measured at 25˚C +/- 0.1 ˚C. Influence of DTT on cutinase activity B. Effect of a reducing agent on cutinase catalytic activity 30μM cutinase was incubated with 5.1 mM DTT for different periods of time. The result of the experiment following the time-dependent cutinase activity towards tributyrin in the presence of DTT is displayed in Figure 17B. It can be seen that cutinase in the absence of DTT retains the specific activity for more than 26 hours. In the presence of DTT in the ratio 30μM cutinase to 5.1 mM DTT, the cutinase specific activity decreased to 41% of the initial activity during 45 minutes of incubation. After 4 hours of incubation the specific activity was 21 % of the initial activity.

## Claims

**1.** A method of coupling a disulfide bridge containing protein or peptide to a carrier comprising the following steps,

a) irradiating the protein or peptide to create a thiol group in the protein or peptide by disulfide bridge disruption, and
b) incubating the irradiated protein or peptide with a carrier capable of binding a thiol group and thereby obtaining a coupling,

or

a) incubating the protein or peptide with a carrier capable of binding a thiol group, and
b) irradiating the protein or peptide in the presence of said carrier to create a thiol group in the protein or peptide by

disulfide bridge disruption and thereby obtaining a coupling wherein the carrier is a support whereon more than one disulfide-bridge-containing protein or peptide are coupled, each protein or peptide being coupled to said carrier through said created thiol group.

**2.** A method according to claim 1, wherein the protein or peptide comprises more than one disulfide bridge.

**3.** A method according to any one of claims 1 or 2, wherein said irradiation step comprises light of a wavelength that excites one or more aromatic amino acids.

**4.** A method according to any one of claims 1 to 3, wherein said irradiation step comprises light of a wavelength that excites one specific aromatic amino acid.

**5.** A method according to any one of claims 1 to 4, wherein said aromatic amino acid(s) is/are selected from tryptophan, tyrosine and phenylalanine.

**6.** A method according to any one of claims 1 to 5, wherein the irradiation is performed by multi-photon excitation, preferably by two-photon excitation.

**7.** A method according to any one of claims 1 to 5, wherein said irradiation comprises light with a wavelength of about 295nm, 275nm or 254nm.

**8.** A method according to claim 5, wherein said aromatic amino acid is tryptophan.

**9.** A method according to claim 7, wherein the wavelength is about 295nm.

**10.** A method according to any one of claims 2 to 9, further comprising the steps of:

a) verifying one or more disulfide bridges in said protein or peptide,
b) identifying one or more aromatic amino acid residues being a spatial neighbour of said one or more disulfide bridges, for the transfer of excitation energy from said one or more aromatic amino acid to said one or more disulfide bridges,
c) selecting a wavelength which specifically excites one or more of said aromatic amino acid residues, thereby disrupting one or more of said disulfide bonds.

**11.** A method according to claim 10 wherein the aromatic amino acid residue is within 10Å of the disulfide bridge

**12.** A method according to claim 11, wherein the plane of the dipole of the side-chain of the aromatic amino acid is not orthogonal to the plane of the disulfide bridge.

**13.** A method according to claims 10-12, wherein the amino acid residues located within an 8Å radius of the indole ring of said aromatic amino acid residue are over-represented by amidic amino acid residues (Asn, Gln), as well as, short aliphatic amino acid residues (Gly, Ala, Val) and /or long aliphatic amino acid residues (Leu, Ile) by at least 1 fold, and under-represented by charged amino acids (His, Lys, Arg)(Asp, Glu) and proline residues by at least 1 fold.

**14.** A method according to any one of claims 1 to 9, wherein said protein or peptide is irradiated in the presence of a free aromatic amino acid.

**15.** A method according to any one of claims 1 to 14, wherein said coupling is an immobilization on said support.

**16.** A method according to claim 15, wherein said immobilization is spatially controlled.

**17.** A method according to claim 16, wherein said support is a derivatised support that is capable of binding a thiol group.

**18.** A method according to claim 17, wherein said support comprises a thiol group or a disulfide bridge.

**19.** A method according to claim 18, wherein the support comprises a spacer.

**20.** A method according to any one of claims 1 to 19, wherein the protein or peptide can furthermore be released from the carrier by irradiating the protein or peptide to create a thiol group in the protein or peptide by disulfide bridge disruption.

**21.** A method according to claim 17, wherein said support comprises gold.

**22.** A method according to any of the preceding claims wherein the coupling is limited to the focal point(s) of illumination.

**23.** A method according to claim 22, wherein the focal spot is 1 micrometer or less.

**24.** A support comprising more than one protein or peptide coupled by the method of any one of claims 1 to 23.

**25.** A support according to claim 24, wherein the coupled protein or peptide are specifically oriented.

**26.** A support according to claim 24 or 25, wherein the support is selected from the group consisting of a particle, electronic chip, slide, wafer, resign, well, tube, microarray and membrane.

**27.** A support according to any of the claims 24-26, wherein the support comprises a material selected from the group consisting of topaz, polystyrene, polyethylene, polyester, polyethermide, polypropylene, polycarbonate, polysulfone, polymethylmethacrylate, poly(vinylidene fluoride), silicone, diamond, quartz and silica, silicium, metal, nylon, nitrocellulose, agarose, cellulose and ceramic.

**28.** A support according to any one of claims 24 to 27, wherein the protein or peptide coupled to the support are spatially controlled.

**29.** A support according to any of the claims 24-28, wherein the coupling is limited to the focal point(s) of illumination

**30.** Use of a support according to any one of claims 24 to 29 for a bio-functional reaction.

**31.** Use according to claim 30, wherein said bio-functional reaction is selected from the group consisting of a bio-sensor, chromatography, immunodetection, enzyme assay, nucleotide binding detection, protein-protein interaction, protein modification, carrier targeting and protein targeting.

**32.** Use of the method according to any one of claims 1 to 23 for the production of a bio-sensor or a protein/peptide microarray.

**33.** Use according to claim 30 or 31 in a diagnostic or biosensor kit.

**Patentansprüche**

**1.** Verfahren zum Koppeln eines eine Disulfidbrücke enthaltenden Proteins oder Peptids an einen Träger, umfassend die folgenden Schritte

a) Bestrahlen des Proteins oder Peptids, um durch Spalten einer Disulfidbrücke eine Thiolgruppe in dem Protein oder Peptid zu erzeugen, und
b) Inkubieren des bestrahlten Proteins oder Peptids mit einem Träger, der im Stande ist, eine Thiolgruppe zu binden, und **dadurch** Erhalten einer Kopplung,

oder

a) Inkubieren des Proteins oder Peptids mit einem Träger, der im Stande ist, eine Thiolgruppe zu binden, und
b) Bestrahlen des Proteins oder Peptids in Gegenwart des Trägers, um durch Spalten einer Disulfidbrücke eine Thiolgruppe in dem Protein oder Peptid zu erzeugen, und **dadurch** Erhalten einer Kopplung

wobei der Träger ein Trägermaterial ist, auf dem mehr als ein eine Disulfidbrücke enthaltendes Protein oder Peptid gekoppelt sind, wobei jedes Protein oder Peptid über die erzeugte Thiolgruppe an den Träger gekoppelt ist.

**2.** Verfahren nach Anspruch 1, wobei das Protein oder Peptid mehr als eine Disulfidbrücke umfasst.

**3.** Verfahren nach einem der Ansprüche 1 oder 2, wobei der Bestrahlungsschritt Licht mit einer Wellenlänge umfasst, welche eine oder mehrere aromatische Aminosäuren anregt.

**4.** Verfahren nach einem der Ansprüche 1 bis 3, wobei der Bestrahlungsschritt Licht mit einer Wellenlänge umfasst, welche eine spezifische aromatische Aminosäure anregt.

**5.** Verfahren nach einem der Ansprüche 1 bis 4, wobei die aromatische(n) Aminosäure(n) aus Tryptophan, Tyrosin und Phenylalanin ausgewählt ist/sind.

**6.** Verfahren nach einem der Ansprüche 1 bis 5, wobei die Bestrahlung durch Multiphotonenanregung, vorzugsweise durch Zweiphotonenanregung erfolgt.

**7.** Verfahren nach einem der Ansprüche 1 bis 5, wobei die Bestrahlung Licht mit einer Wellenlänge von ungefähr 295 nm, 275 nm oder 254 nm umfasst.

**8.** Verfahren nach Anspruch 5, wobei die aromatische Aminosäure Tryptophan ist.

**9.** Verfahren nach Anspruch 7, wobei die Wellenlänge ungefähr 295 nm beträgt.

**10.** Verfahren nach einem der Ansprüche 2 bis 9, außerdem umfassend die Schritte:

a) Verifizieren von einer oder mehreren Disulfidbrücken in dem Protein oder Peptid,
b) Identifizieren von einem oder mehreren aromatischen Aminosäureresten, welcher bzw. welche ein räumlicher Nachbar der einen oder mehreren Disulfidbrücken ist bzw. sind, für die Übertragung von Anregungsenergie von der einen oder mehreren aromatischen Aminosäure auf die eine oder mehreren Disulfidbrücken,
c) Auswählen einer Wellenlänge, welche einen oder mehrere der aromatischen Aminosäurereste spezifisch anregt, wodurch eine oder mehrere der Disulfidbindungen gespalten wird bzw. werden.

**11.** Verfahren nach Anspruch 10, wobei der aromatische Aminosäurerest sich innerhalb von 10 Å von der Disulfidbrücke befindet.

**12.** Verfahren nach Anspruch 11, wobei die Ebene des Dipols der Seitenkette der aromatischen Aminosäure nicht orthogonal zu der Ebene der Disulfidbrücke ist.

**13.** Verfahren nach den Ansprüchen 10-12, wobei die innerhalb eines 8 Å-Radius von dem Indolring des aromatischen Aminosäurerests befindlichen Aminosäurereste durch amidische Aminosäurereste (Asn, Gln) sowie kurze aliphatische Aminosäurereste (Gly, Ala, Val) und/oder lange aliphatische Aminosäurereste (Leu, Ile) um mindestens das 1-fache überrepräsentiert sind und durch geladene Aminosäuren (His, Lys, Arg), (Asp, Glu) und Prolinreste um mindestens das 1-fache unterrepräsentiert sind.

**14.** Verfahren nach einem der Ansprüche 1 bis 9, wobei das Protein oder Peptid in Gegenwart einer freien aromatischen Aminosäure bestrahlt wird.

**15.** Verfahren nach einem der Ansprüche 1 bis 14, wobei es sich bei der Kopplung um eine Immobilisierung auf dem Trägermaterial handelt.

**16.** Verfahren nach Anspruch 15, wobei die Immobilisierung räumlich kontrolliert ist.

**17.** Verfahren nach Anspruch 16, wobei das Trägermaterial ein derivatisiertes Trägermaterial ist, welches eine Thiolgruppe binden kann.

**18.** Verfahren nach Anspruch 17, wobei das Trägermaterial eine Thiolgruppe oder eine Disulfidbrücke umfasst.

**19.** Verfahren nach Anspruch 18, wobei das Trägermaterial einen Abstandshalter (Spacer) umfasst.

**20.** Verfahren nach einem der Ansprüche 1 bis 19, wobei das Protein oder Peptid außerdem durch Bestrahlen des Proteins oder Peptids zum Erzeugen einer Thiolgruppe in dem Protein oder Peptid durch Spalten einer Disulfidbrücke von dem Träger freigesetzt werden kann.

**21.** Verfahren nach Anspruch 17, wobei das Trägermaterial Gold umfasst.

**22.** Verfahren nach einem der vorangehenden Ansprüche, wobei die Kopplung auf den Brennpunkt bzw. die Brennpunkte der Belichtung beschränkt ist.

**23.** Verfahren nach Anspruch 22, wobei der Brennfleck 1 Mikrometer oder weniger beträgt.

**24.** Trägermaterial, umfassend mehr als ein Protein oder Peptid, gekoppelt durch das Verfahren nach einem der Ansprüche 1 bis 23.

**25.** Trägermaterial nach Anspruch 24, wobei das gekoppelte Protein oder Peptid spezifisch ausgerichtet ist.

**26.** Trägermaterial nach Anspruch 24 oder 25, wobei das Trägermaterial ausgewählt ist aus der Gruppe bestehend aus einem Teilchen, elektronischen Chip, Objektglas bzw. Objektträger, Wafer, Harz, einer Vertiefung (Well), einem Rohr, Mikroarray und einer Membran.

**27.** Trägermaterial nach einem der Ansprüche 24-26, wobei das Trägermaterial ein Material umfasst, das ausgewählt ist aus der Gruppe bestehend aus Topas, Polystyrol, Polyethylen, Polyester, Polyethermid, Polypropylen, Polycarbonat, Polysulfon, Polymethylmethacrylat, Polyvinylidenfluorid, Silicon, Diamant, Quarz und Siliciumdioxid, Silicium, Metall, Nylon, Nitrocellulose, Agarose, Cellulose und Keramik.

**28.** Trägermaterial nach einem der Ansprüche 24 bis 27, wobei das an das Trägermaterial gekoppelte Protein oder Peptid räumlich kontrolliert ist.

**29.** Trägermaterial nach einem der Ansprüche 24-28, wobei die Kopplung auf den Brennpunkt bzw. die Brennpunkte der Belichtung beschränkt ist.

**30.** Verwendung eines Trägermaterials nach einem der Ansprüche 24 bis 29 für eine biofunktionelle Reaktion.

**31.** Verwendung nach Anspruch 30, wobei die biofunktionelle Reaktion ausgewählt ist aus der Gruppe bestehend aus einem Biosensor, Chromatografie, Immunnachweis, Enzymtest, Nucleotidbindungsnachweis, Protein-Protein-Wechselwirkung, Proteinmodifizierung, Träger-Targeting und Protein-Targeting.

**32.** Verwendung des Verfahrens nach einem der Ansprüche 1 bis 23 zum Herstellen eines Biosensors oder eines Protein/Peptid-Mikroarrays.

**33.** Verwendung nach Anspruch 30 oder 31 in einem diagnostischen oder Biosensor-Kit.

## Revendications

**1.** Procédé de couplage d’une protéine ou d’un peptide contenant un pont disulfure à un support, comprenant les étapes suivantes :

a) irradiation de la protéine ou du peptide pour créer un groupe thiol dans la protéine ou le peptide par la rupture du pont disulfure, et
b) incubation de la protéine ou du peptide irradié(e) avec un support capable de lier un groupe thiol et par conséquent d’obtenir un couplage,

ou

a) incubation de la protéine ou du peptide avec un support capable de lier un groupe thiol, et
b) irradiation de la protéine ou du peptide en présence dudit support pour créer un groupe thiol dans la protéine ou dans le peptide par rupture du pont disulfure et par conséquent obtenir un couplage,

dans lequel le support est un support sur lequel sont couplées plus d’une protéine ou d’un peptide contenant un pont disulfure, chaque protéine ou peptide étant couplé(e) audit support par ledit groupe thiol créé.

**2.** Procédé selon la revendication 1, dans lequel la protéine ou le peptide comprend plus d'un pont disulfure.

**3.** Procédé selon l'une quelconque des revendications 1 ou 2, dans lequel l'étape d'irradiation comprend de la lumière d'une longueur d'onde qui excite un ou plusieurs acides aminés aromatiques.

**4.** Procédé selon l'une quelconque des revendications 1 à 3, dans lequel ladite étape d'irradiation comprend de la lumière d'une longueur d'onde qui excite un acide aminé aromatique spécifique.

**5.** Procédé selon l'une quelconque des revendications 1 à 4, dans lequel ledit ou lesdits acides aminés aromatiques est/sont sélectionné(s) parmi le tryptophane, la trypsine et la phénylalanine.

**6.** Procédé selon l'une quelconque des revendications 1 à 5, dans lequel l'irradiation est réalisée par excitation multiphotons, de préférence par une excitation à deux photons.

**7.** Procédé selon l'une quelconque des revendications 1 à 5, dans lequel ladite irradiation comprend une lumière ayant une longueur d'onde d'environ 295 nm, 275 nm ou 254 nm.

**8.** Procédé selon la revendication 5, dans lequel ledit acide aminé aromatique est le tryptophane.

**9.** Procédé selon la revendication 7, dans lequel la longueur d'onde est d'environ 295 nm.

**10.** Procédé selon l'une quelconque des revendications 2 à 9, comprenant en outre les étapes consistant à :

a) vérifier un ou plusieurs ponts disulfure dans ladite protéine ou ledit peptide,
b) identifier un ou plusieurs résidus acide aminé aromatique comme étant un voisin spatial dudit un ou desdits ponts disulfure, pour le transfert de l'énergie d'excitation depuis ledit un ou lesdits plusieurs acides aminés aromatiques vers ledit un ou lesdits plusieurs ponts disulfure,
c) sélectionner une longueur d'onde qui excite spécifiquement un ou plusieurs desdits résidus acide aminé aromatique, rompant de ce fait un ou plusieurs desdits ponts disulfure.

**11.** Procédé selon la revendication 10, dans lequel le résidu acide aminé aromatique est dans les 10 Å du pont disulfure.

**12.** Procédé selon la revendication 11, dans lequel le plan du dipôle de la chaîne latérale de l'acide aminé aromatique n'est pas orthogonal au plan du pont disulfure.

**13.** Procédé selon les revendications 10 à 12, dans lequel les résidus acide aminé situés au sein d'un rayon de 8 Å de l'anneau indole dudit résidu acide aminé aromatique sont sur-représentés par des résidus acide aminé amidique (Asn, Gln), ainsi que des résidus acide aminé aliphatique court (Gly, Ala, Val) et/ou des résidus acide aminé aliphatique long (Leu, Ile) d'au moins un pli, et sous-représentés par des acides aminés chargés (His, Lys, Arg) (Asp, Glu) et des résidus proline d'au moins un pli.

**14.** Procédé selon l'une quelconque des revendications 1 à 9, dans lequel ladite protéine ou ledit peptide est irradié(e) en présence d'un acide aminé aromatique libre.

**15.** Procédé selon l'une quelconque des revendications 1 à 14, dans lequel ledit couplage est une immobilisation sur ledit support.

**16.** Procédé selon la revendication 15, dans lequel ladite immobilisation est contrôlée spatialement.

**17.** Procédé selon la revendication 16, dans lequel ledit support est un support dérivé capable de lier un groupe thiol.

**18.** Procédé selon la revendication 17, dans lequel ledit support comprend un groupe thiol ou un pont disulfure.

**19.** Procédé selon la revendication 18, dans lequel le support comprend un espaceur.

**20.** Procédé selon l'une quelconque des revendications 1 à 19, dans lequel la protéine ou le peptide peut de plus être libéré(e) du support par irradiation de la protéine ou du peptide pour créer un groupe thiol dans la protéine ou le peptide par une rupture du pont disulfure.

**21.** Procédé selon la revendication 17, dans lequel ledit support comprend de l'or.

**22.** Procédé selon l'une quelconque des revendications précédentes, dans lequel le couplage est limité au point focal d'illumination.

**23.** Procédé selon la revendication 22, dans lequel la tache focale est de 1 micron ou moins.

**24.** Support comprenant plus d'une protéine ou d'un peptide couplé(e)s par le procédé selon l'une quelconque des revendications 1 à 23.

**25.** Support selon la revendication 24, dans lequel la protéine ou le peptide couplé(e) est orienté(e) de façon spécifique.

**26.** Support selon la revendication 24 ou 25, dans lequel le support est sélectionné dans le groupe composé d'une particule, d'une puce électronique, d'une lamelle, d'une pastille, d'une résine, d'un puits, d'un tube, d'une puce à ADN et d'une membrane.

**27.** Support selon l'une quelconque des revendications 24 à 26, dans lequel le support comprend un matériau sélectionné dans le groupe composé du topaze, du polystyrène, du polyéthylène, du polyester, du polyétherimide, du polypropylène, du polycarbonate, de la polysulfone, du méthacrylate de polyméthyle, du poly(fluorure de vinylidène), de la silicone, du diamant, du quartz et de la silice, du silicium, du métal, du nylon, de la nitrocellulose, de l'agarose, de la cellulose et de la céramique.

**28.** Support selon l'une quelconque des revendications 24 à 27, dans lequel la protéine ou le peptide couplé(e) au support est contrôlé(e) spatialement.

**29.** Support selon l'une quelconque des revendications 24 à 28, dans lequel le couplage est limité au(x) point(s) focal (ux) d'illumination.

**30.** Utilisation d'un support selon l'une quelconque des revendications 24 à 29 pour une réaction biofonctionnelle.

**31.** Utilisation selon la revendication 30, dans lequel ladite réaction biofonctionnelle est sélectionnée dans le groupe composé d'un biodétecteur, d'une chromatographie, d'une immunodétection, d'une analyse enzymatique, d'une détection de liaison de nucléotide, d'une interaction protéine-protéine, d'une modification de protéine, d'un ciblage de support et d'un ciblage de protéine.

**32.** Utilisation du procédé selon l'une quelconque des revendications 1 à 23 pour la production d'un biodétecteur ou d'une puce à protéines/peptides.

**33.** Utilisation selon la revendication 30 ou 31 dans un kit de diagnostic ou de biodétecteur.

Figure 1

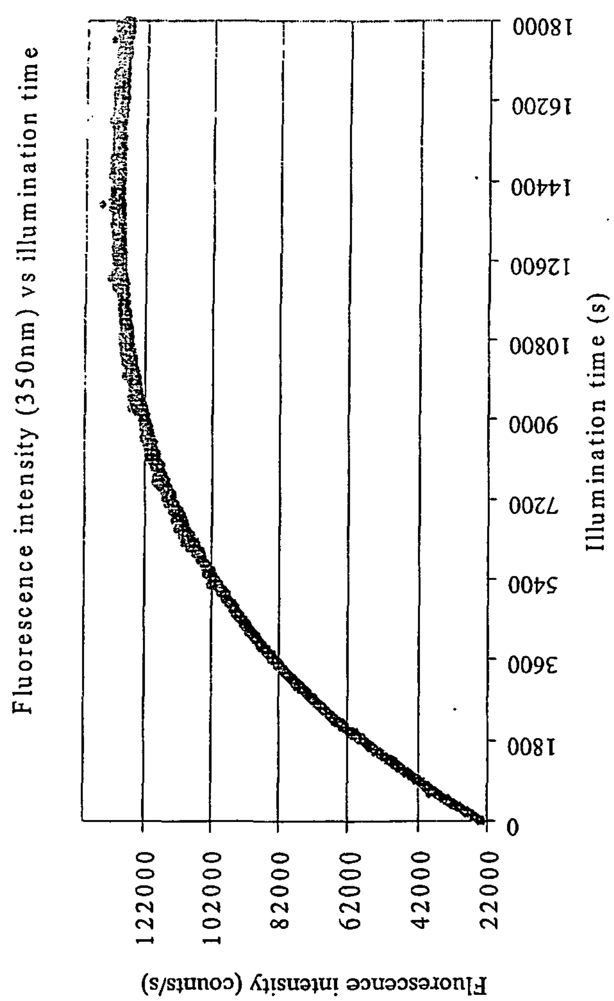
Fluorescence intensity (350nm) vs illumination time
Fluorescence intensity (counts/s)
122000
102000
82000
62000
42000
22000
0
1800
3600
5400
7200
9000
10800
12600
14400
16200
18000
Illumination time (s)

Figure 2

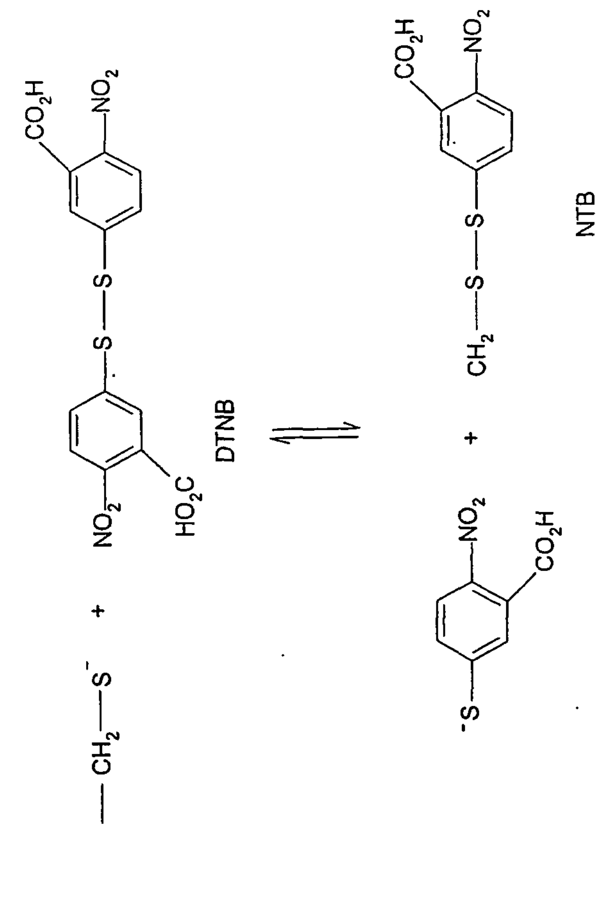

DTNB

NTB

Figure 3

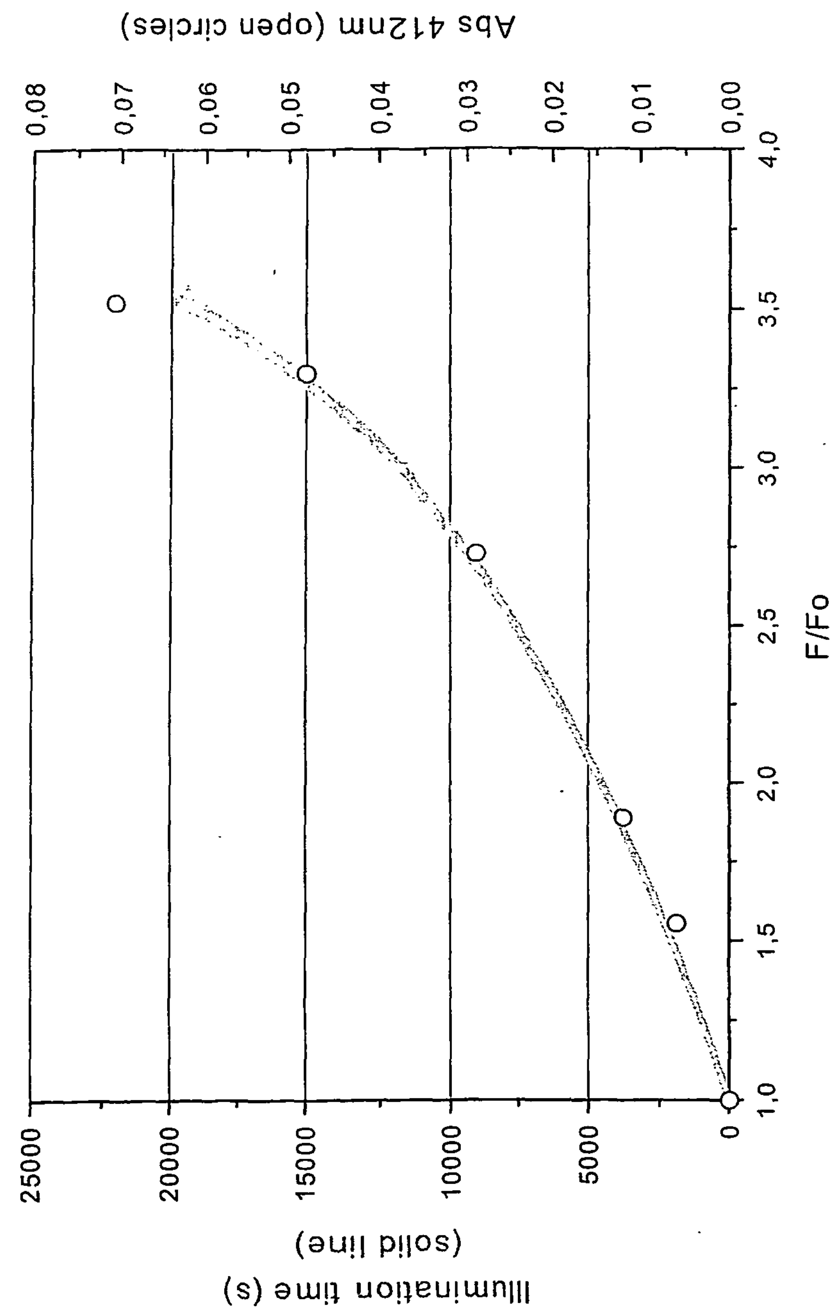
Abs 412nm (open circles)
0,08
0,07
0,06
0,05
0,04
0,03
0,02
0,01
0,00
Illumination time (s)
(solid line)
25000
20000
15000
10000
5000
0
1,0
1,5
2,0
2,5
3,0
3,5
4,0
F/Fo

Figure 4

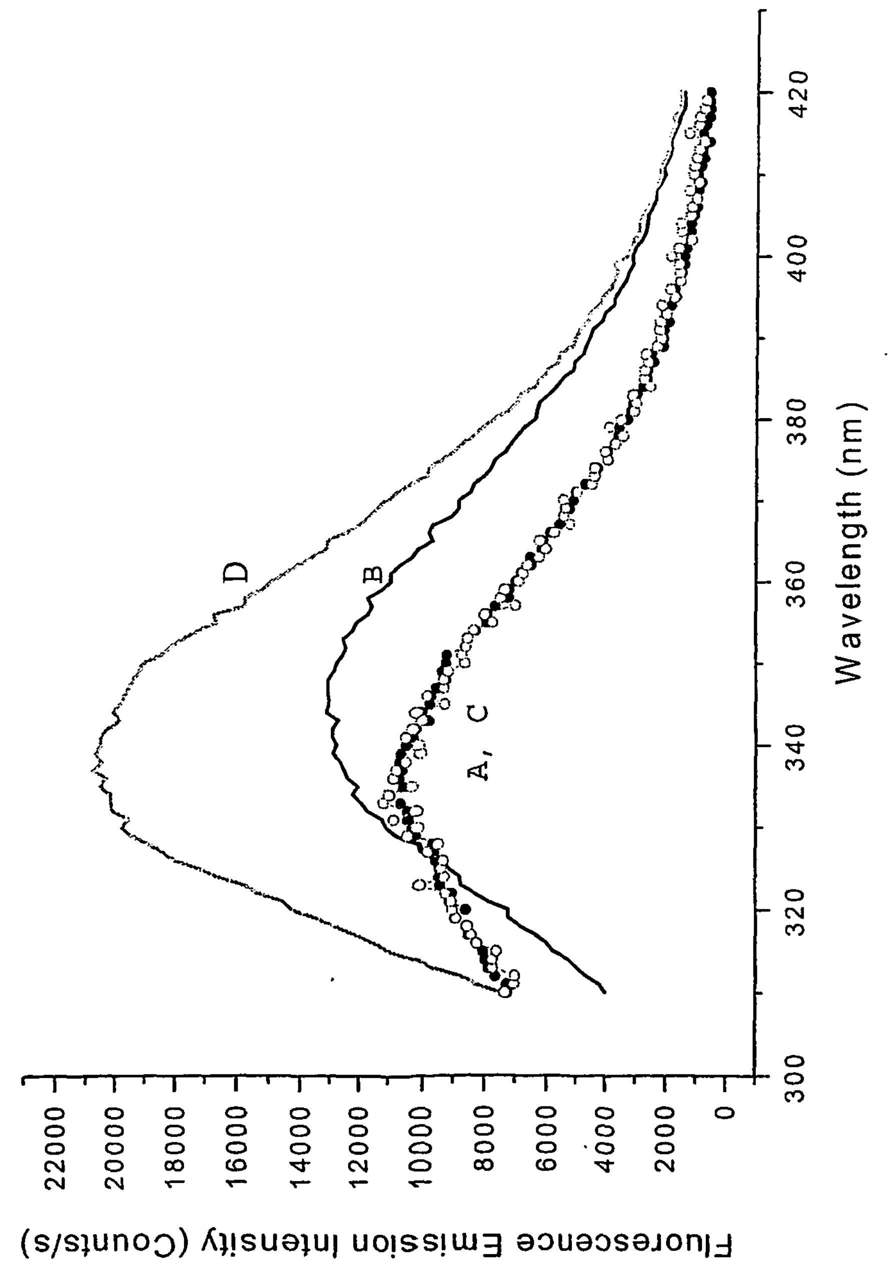
Fluorescence Emission Intensity (Counts/s)
22000
20000
18000
16000
14000
12000
10000
8000
6000
4000
2000
0
300
320
340
360
380
400
420
Wavelength (nm)
D
B
A, C

Figure 5

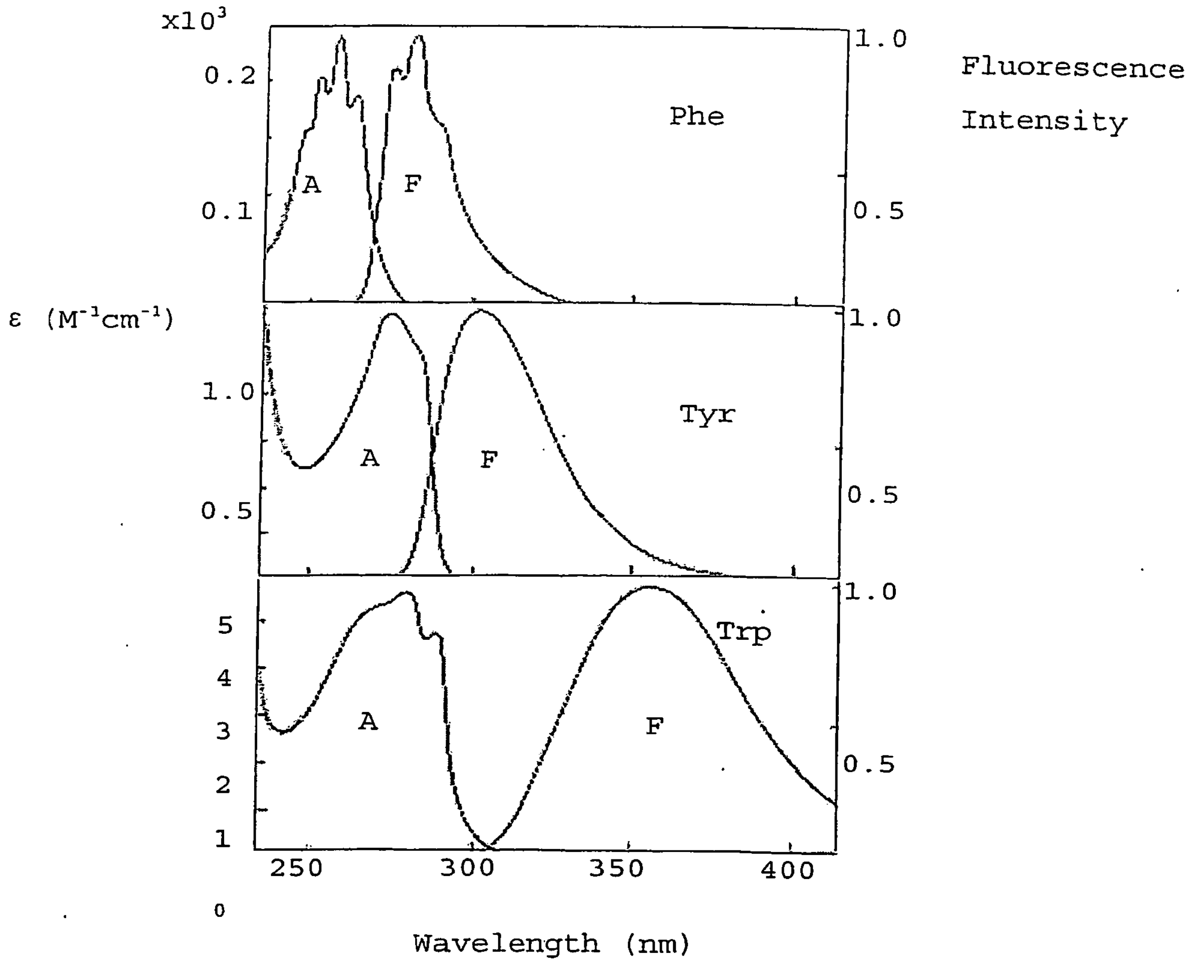
x10³
0.2
0.1
ε (M⁻¹cm⁻¹)
1.0
0.5
5
4
3
2
1
0
A
F
Phe
Tyr
Trp
1.0
0.5
Fluorescence
Intensity
250
300
350
400
Wavelength (nm)

Figure 6

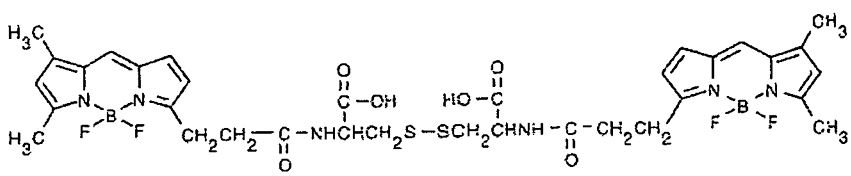

Figure 7

A

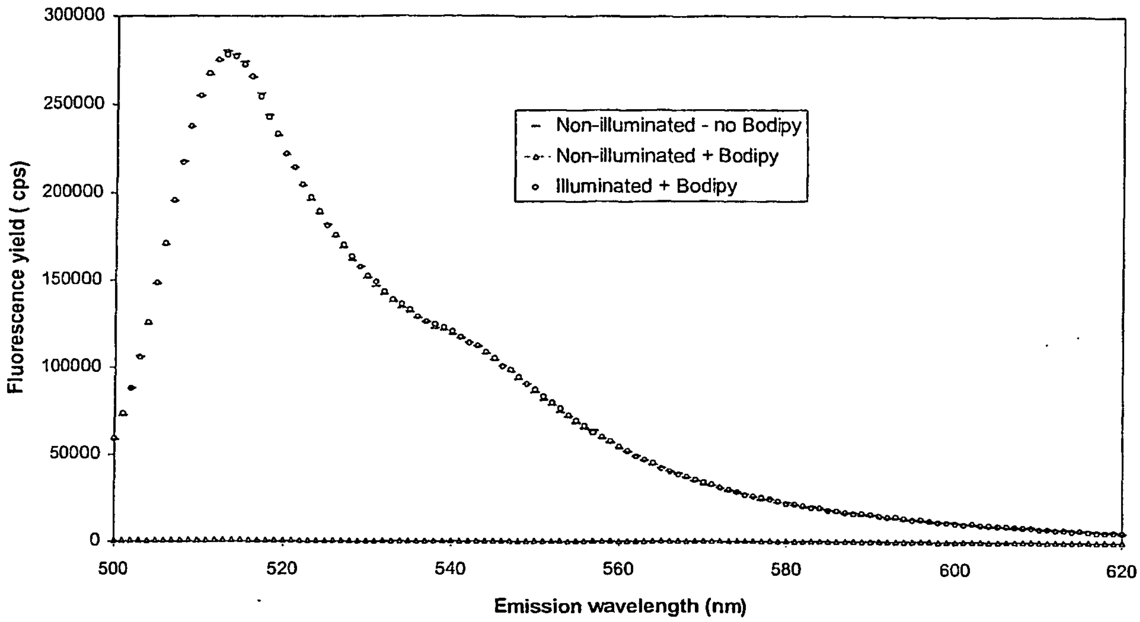
300000
250000
200000
150000
100000
50000
0
500
520
540
560
580
600
620
Fluorescence yield ( cps)
Emission wavelength (nm)
Non-illuminated - no Bodipy
Non-illuminated + Bodipy
Illuminated + Bodipy

B

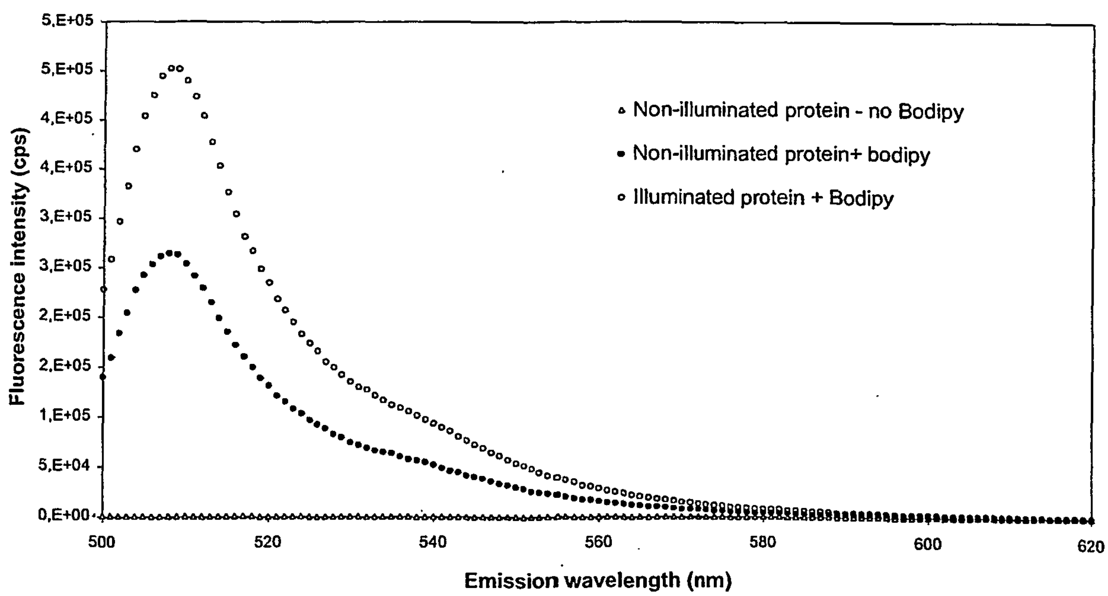
5,E+05
5,E+05
4,E+05
4,E+05
3,E+05
3,E+05
2,E+05
2,E+05
1,E+05
5,E+04
0,E+00
500
520
540
560
580
600
620
Fluorescence intensity (cps)
Emission wavelength (nm)
Non-illuminated protein - no Bodipy
Non-illuminated protein+ bodipy
Illuminated protein + Bodipy

Figure 8

A

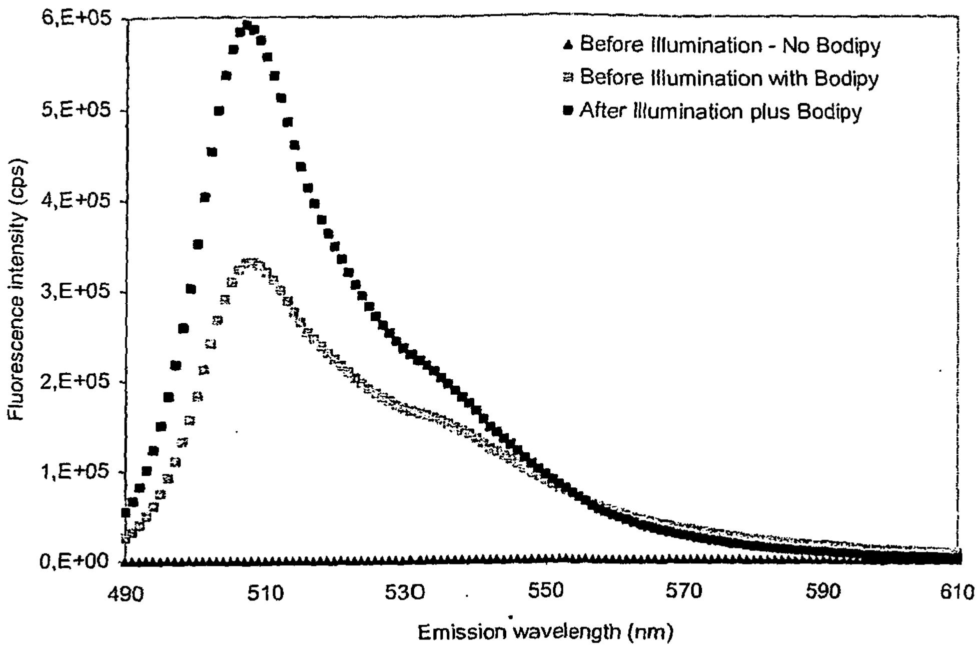
6,E+05
5,E+05
4,E+05
3,E+05
2,E+05
1,E+05
0,E+00
490
510
530
550
570
590
610
Fluorescence intensity (cps)
Emission wavelength (nm)
Before Illumination - No Bodipy
Before Illumination with Bodipy
After Illumination plus Bodipy

B

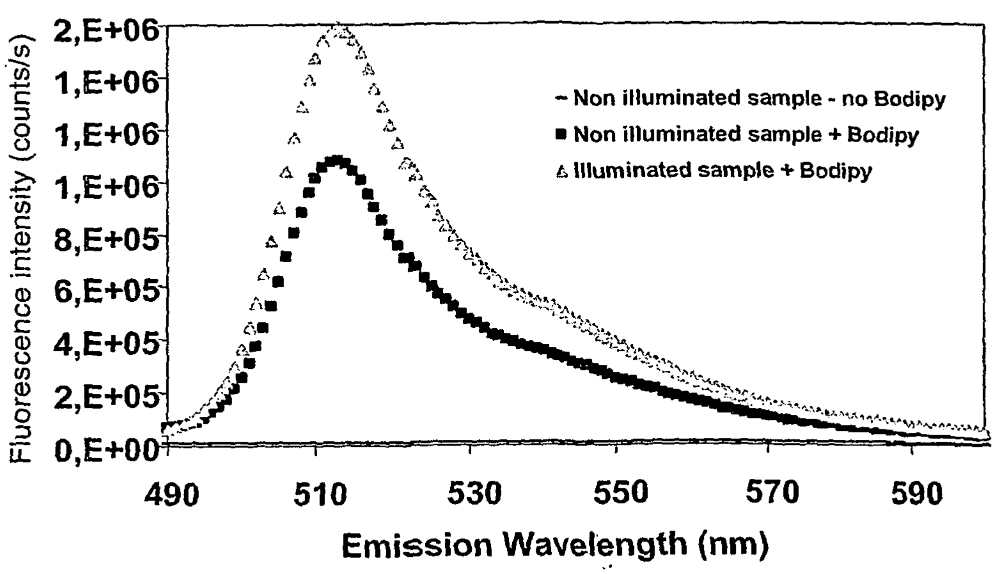
2,E+06
1,E+06
1,E+06
1,E+06
8,E+05
6,E+05
4,E+05
2,E+05
0,E+00
490
510
530
550
570
590
Fluorescence intensity (counts/s)
Emission Wavelength (nm)
Non illuminated sample - no Bodipy
Non illuminated sample + Bodipy
Illuminated sample + Bodipy

C

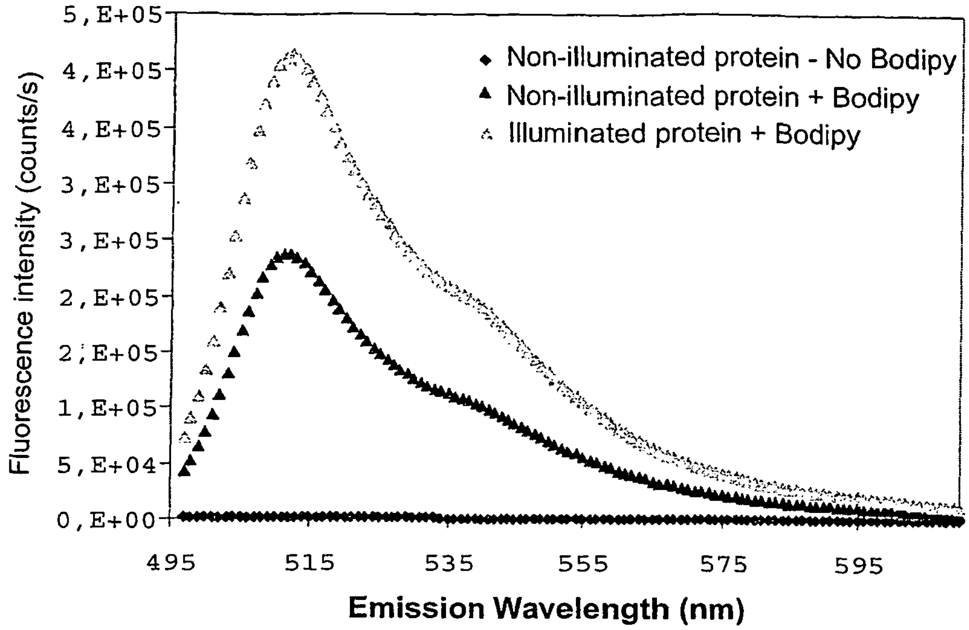
5,E+05
4,E+05
4,E+05
3,E+05
3,E+05
2,E+05
2,E+05
1,E+05
5,E+04
0,E+00
Fluorescence intensity (counts/s)
495
515
535
555
575
595
Emission Wavelength (nm)
Non-illuminated protein - No Bodipy
Non-illuminated protein + Bodipy
Illuminated protein + Bodipy

D

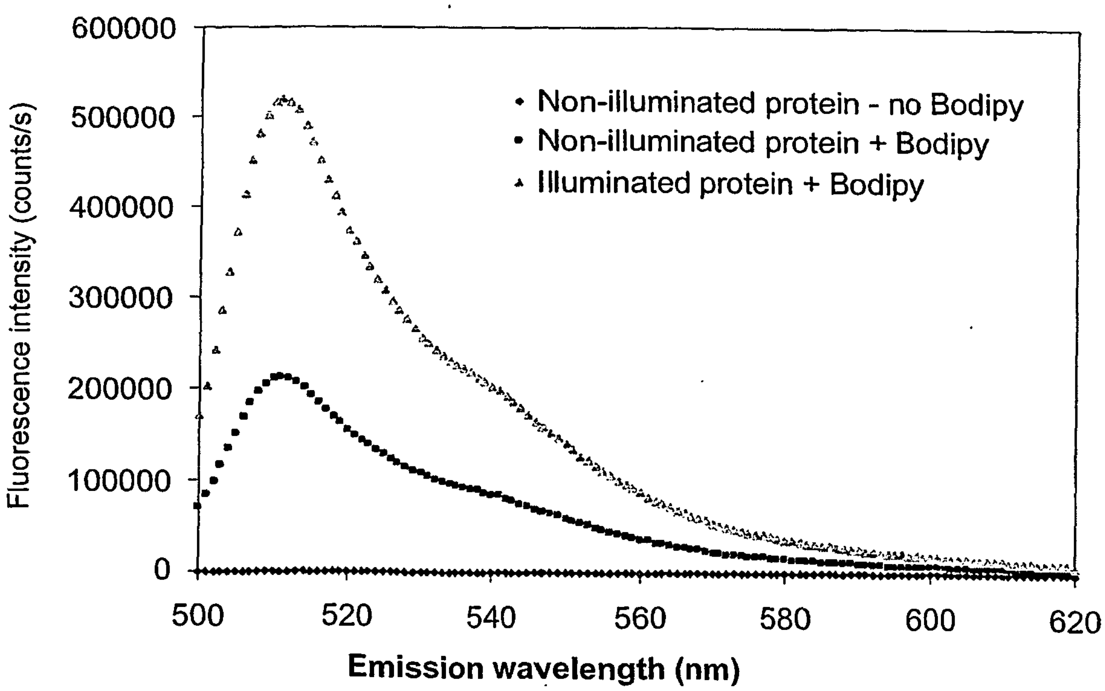
600000
500000
400000
300000
200000
100000
0
Fluorescence intensity (counts/s)
500
520
540
560
580
600
620
Emission wavelength (nm)
Non-illuminated protein - no Bodipy
Non-illuminated protein + Bodipy
Illuminated protein + Bodipy

E

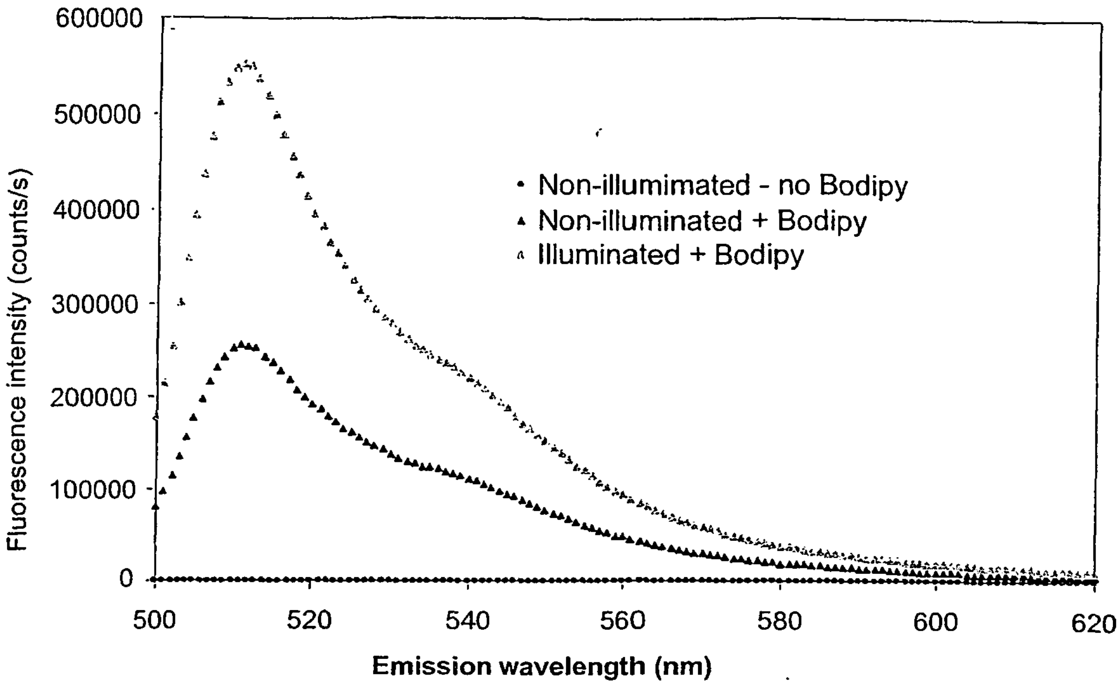
600000
500000
400000
300000
200000
100000
0
Fluorescence intensity (counts/s)
500
520
540
560
580
600
620
Emission wavelength (nm)
Non-illumimated - no Bodipy
Non-illuminated + Bodipy
Illuminated + Bodipy

F

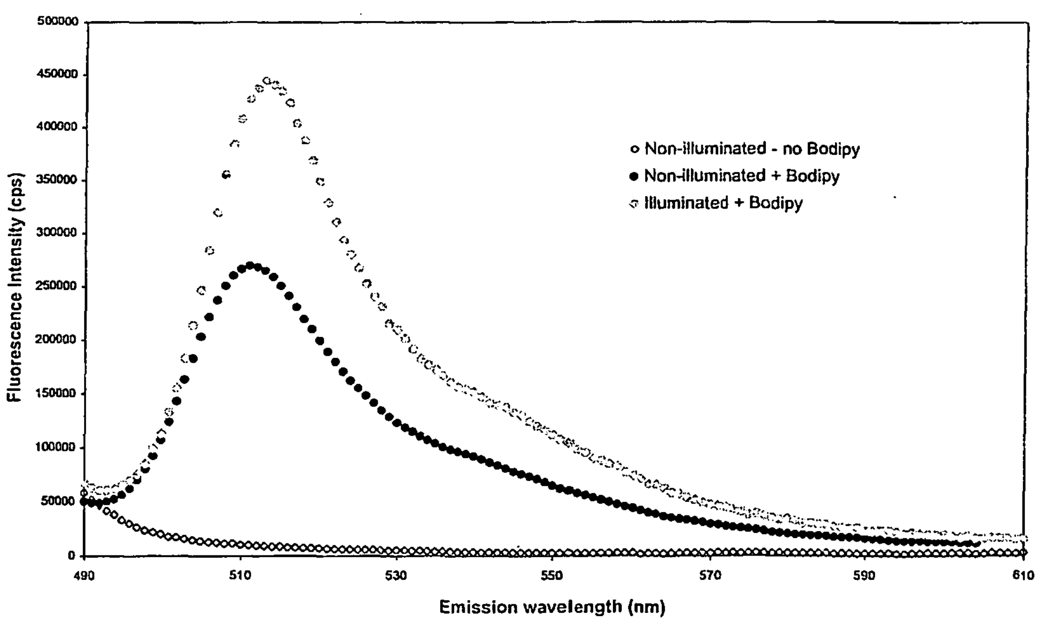
500000
450000
400000
350000
300000
250000
200000
150000
100000
50000
0
Fluorescence Intensity (cps)
490
510
530
550
570
590
610
Emission wavelength (nm)
Non-illuminated - no Bodipy
Non-illuminated + Bodipy
Illuminated + Bodipy

Figure 9

**8 Ångstrøm neighbourhood**

**The "score" is the result of : f(subset)/f(protein)**

**Therefore: "scores" > 1 means that residue/group is overrepresented**

**"scores" < 1 means that residue/group is underrepresented**

- 3,50-4,00
- 3,00-3,50
- 2,50-3,00
- 2,00-2,50
- 1,50-2,00
- 1,00-1,50
- 0,50-1,00
- 0,00-0,50

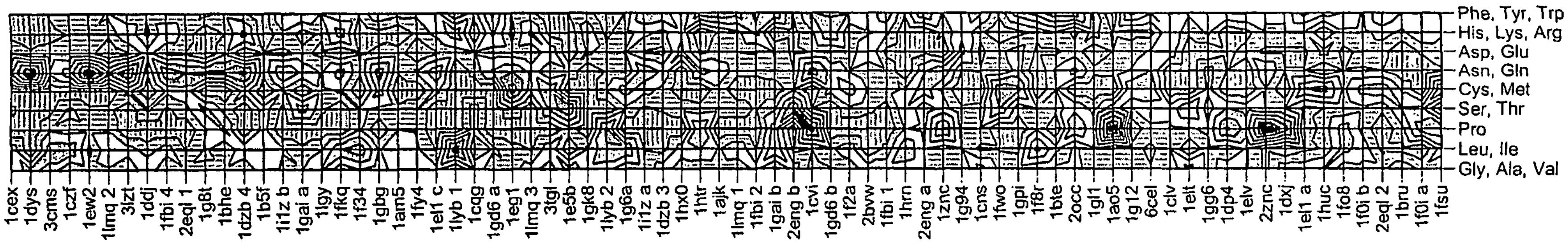

Figure 10

**8 Ångstrøm neighbourhood**

**The "score" is the result of : f(subset)/f(protein)**

**Therefore: "scores" > 1 means that residue/group is overrepresented**
**"scores" < 1 means that residue/group is underrepresented**

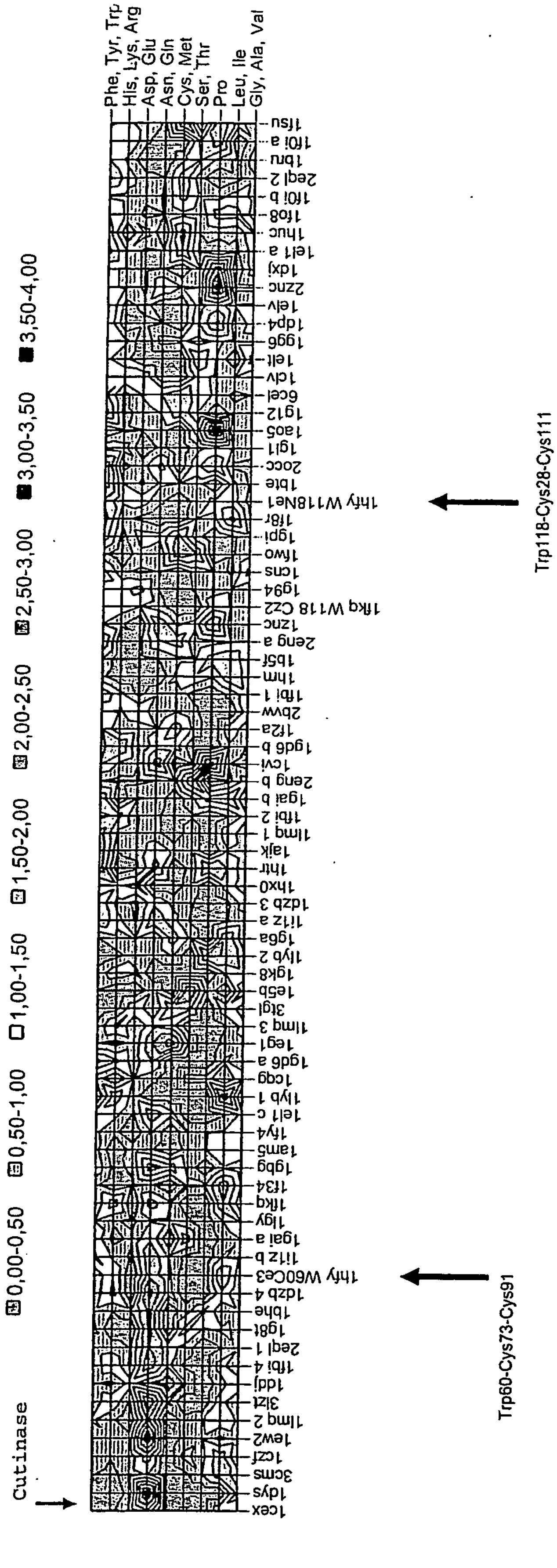

Figure 11

**8 Ångstrøm neighbourhood**
**The triad of cutinase compared to IGG1 triads A)**

**The "score" is the result of : f(subset)/f(protein)**

**Therefore: "scores" > 1 means that residue/group is overrepresented**
**"scores" < 1 means that residue/group is underrepresented**

0,00-0,50 0,50-1,00 1,00-1,50 1,50-2,00 2,00-2,50 2,50-3,00 3,00-3,50 3,50-4,00 4,00-4,50 4,50-5,00 5,00-5,50

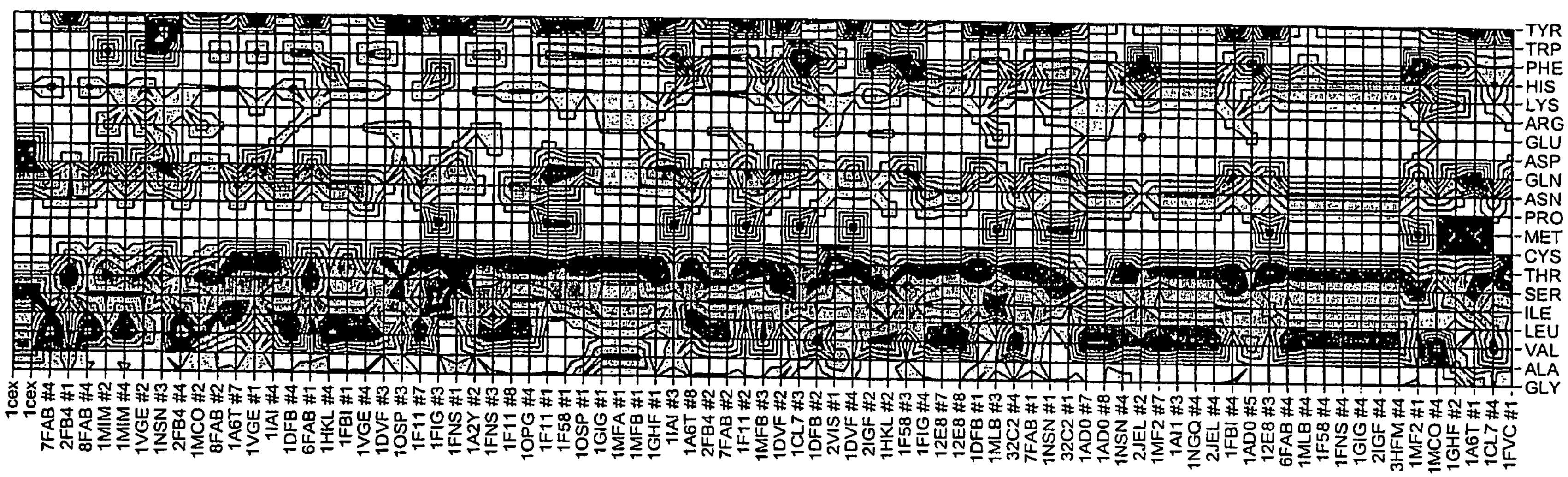

# Figure 12

**8 Ångstrøm neighbourhood**
**The triad of cutinase compared to IGG1 triads B)**

**The "score" is the result of : f(subset)/f(protein)**

**Therefore: "scores" > 1 means that residue/group is overrepresented**
**"scores" < 1 means that residue/group is underrepresented**

0,00-0,50 0,50-1,00 1,00-1,50 1,50-2,00 2,00-2,50 2,50-3,00 3,00-3,50 3,50-4,00 4,00-4,50 4,50-5,00 5,00-5,50 5,50-6,00

6,00-6,50 6,50-7,00 7,00-7,50 7,50-8,00

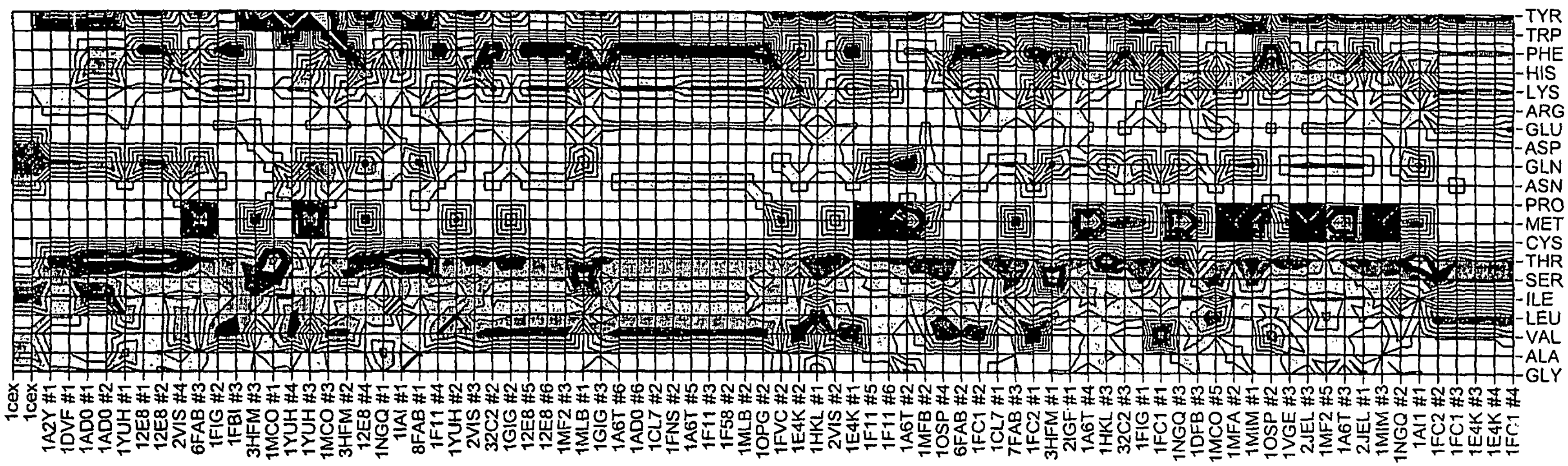

Figure 13

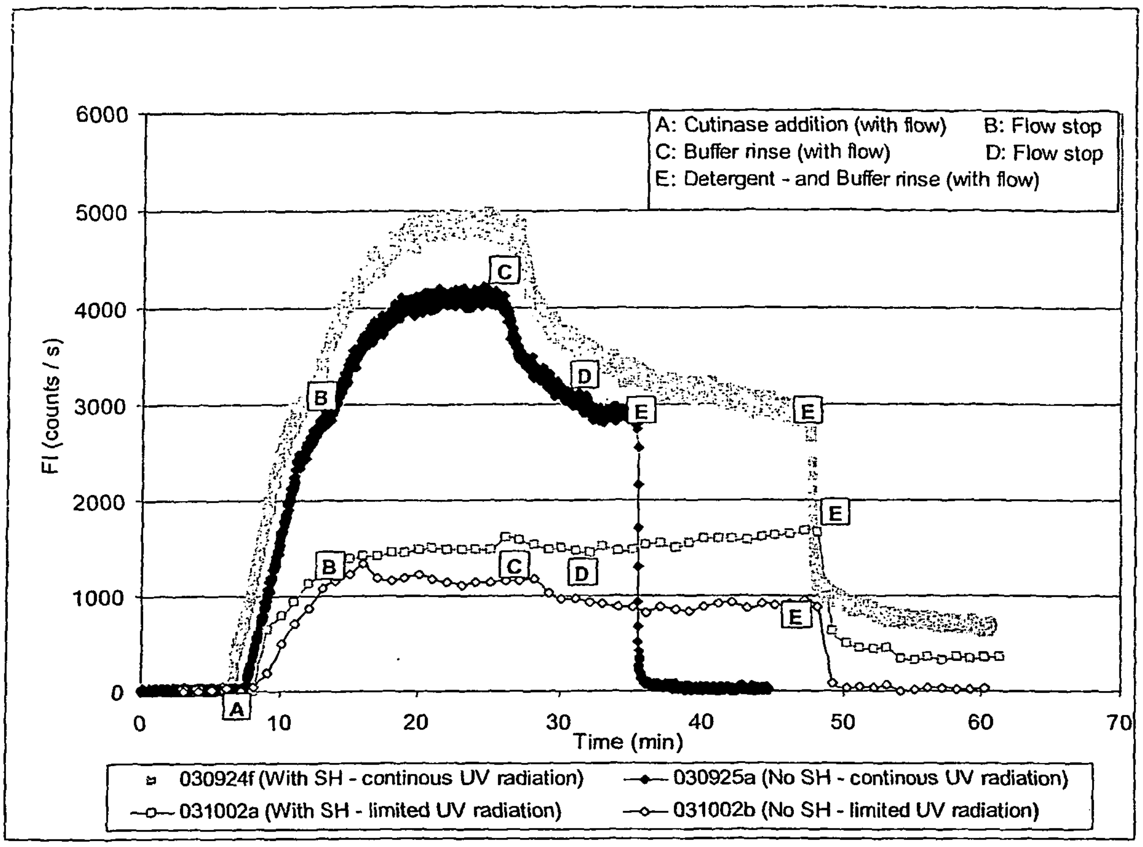

A: Cutinase addition (with flow) B: Flow stop
C: Buffer rinse (with flow) D: Flow stop
E: Detergent - and Buffer rinse (with flow)
FI (counts / s)
Time (min)
0
1000
2000
3000
4000
5000
6000
0
10
20
30
40
50
60
70
A
B
C
D
E
030924f (With SH - continous UV radiation)
030925a (No SH - continous UV radiation)
031002a (With SH - limited UV radiation)
031002b (No SH - limited UV radiation)

Figure 14

A

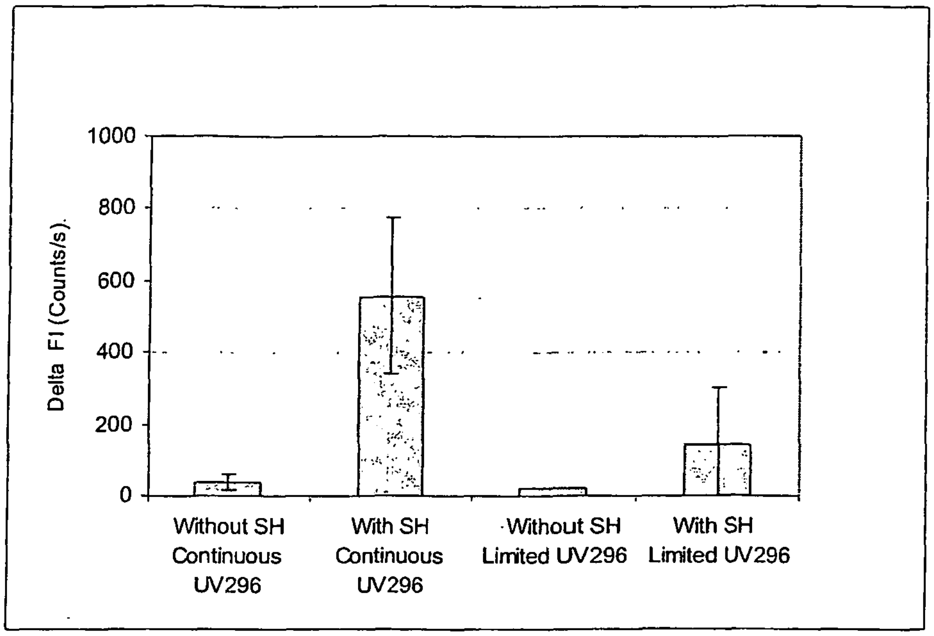
1000
800
600
400
200
0
Delta FI (Counts/s).
Without SH Continuous UV296
With SH Continuous UV296
Without SH Limited UV296
With SH Limited UV296

B

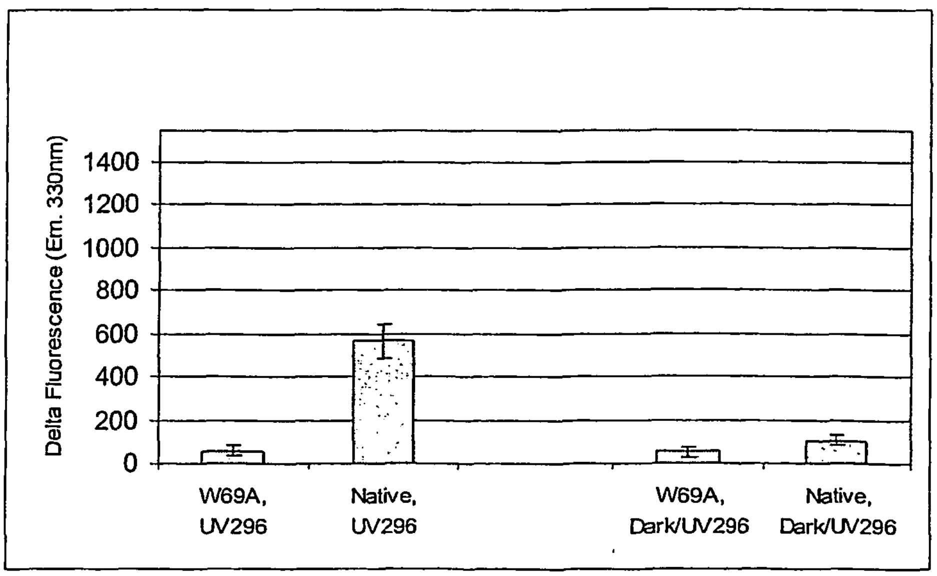
1400
1200
1000
800
600
400
200
0
Delta Fluorescence (Em. 330nm)
W69A, UV296
Native, UV296
W69A, Dark/UV296
Native, Dark/UV296

Figure 15

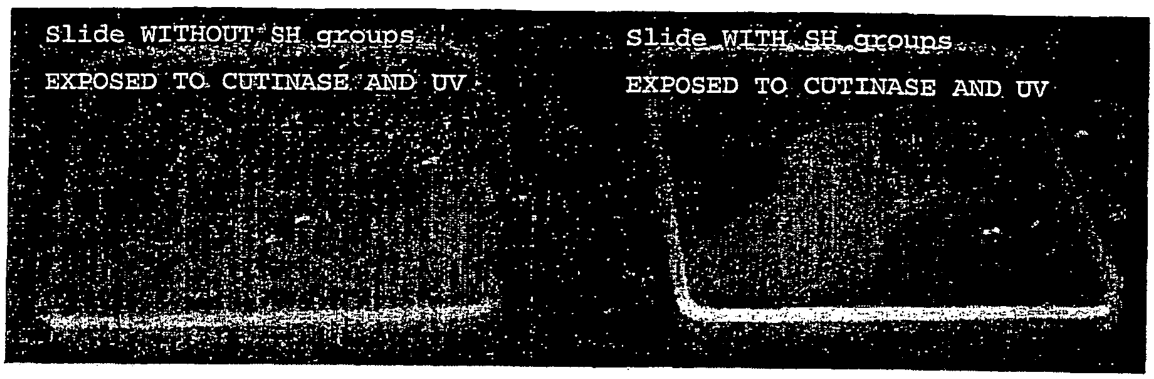
Slide WITHOUT SH groups
EXPOSED TO CUTINASE AND UV
Slide WITH SH groups
EXPOSED TO CUTINASE AND UV

Figure 16

A

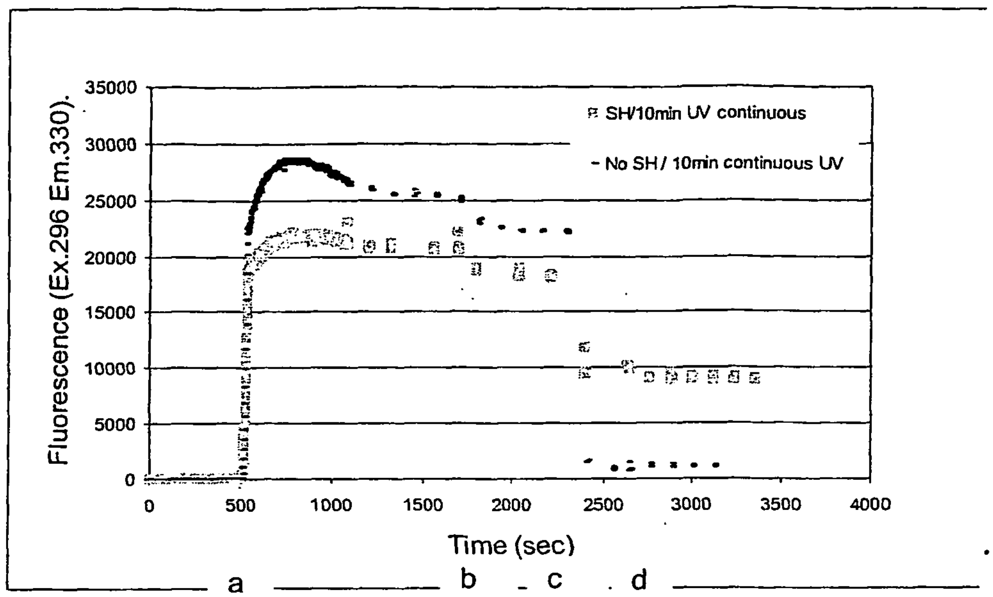
SH/10min UV continuous
No SH / 10min continuous UV
Fluorescence (Ex.296 Em.330).
35000
30000
25000
20000
15000
10000
5000
0
0
500
1000
1500
2000
2500
3000
3500
4000
Time (sec)
a
b
c
d

B

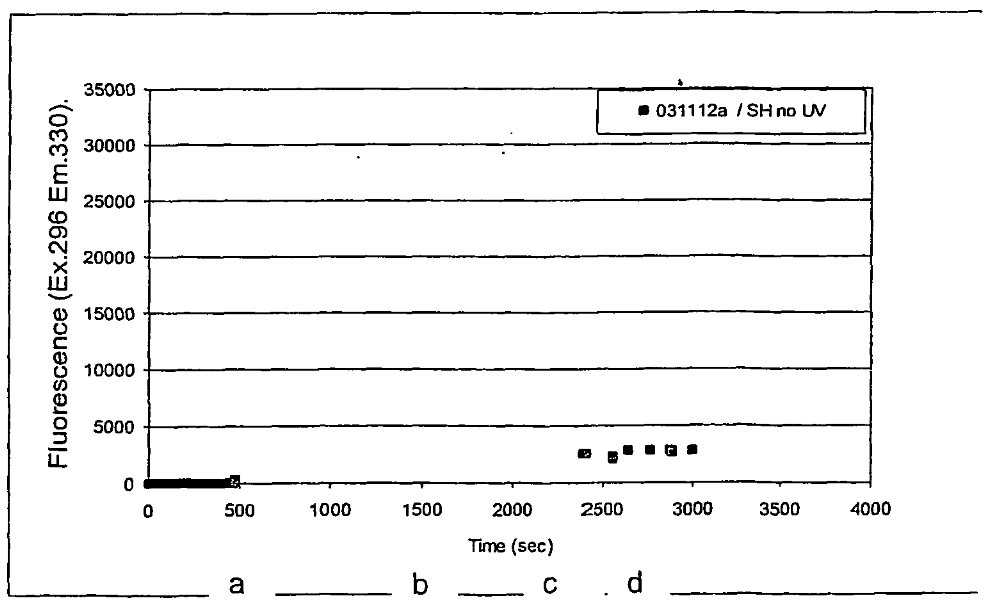
031112a / SH no UV
Fluorescence (Ex.296 Em.330).
35000
30000
25000
20000
15000
10000
5000
0
0
500
1000
1500
2000
2500
3000
3500
4000
Time (sec)
a
b
c
d

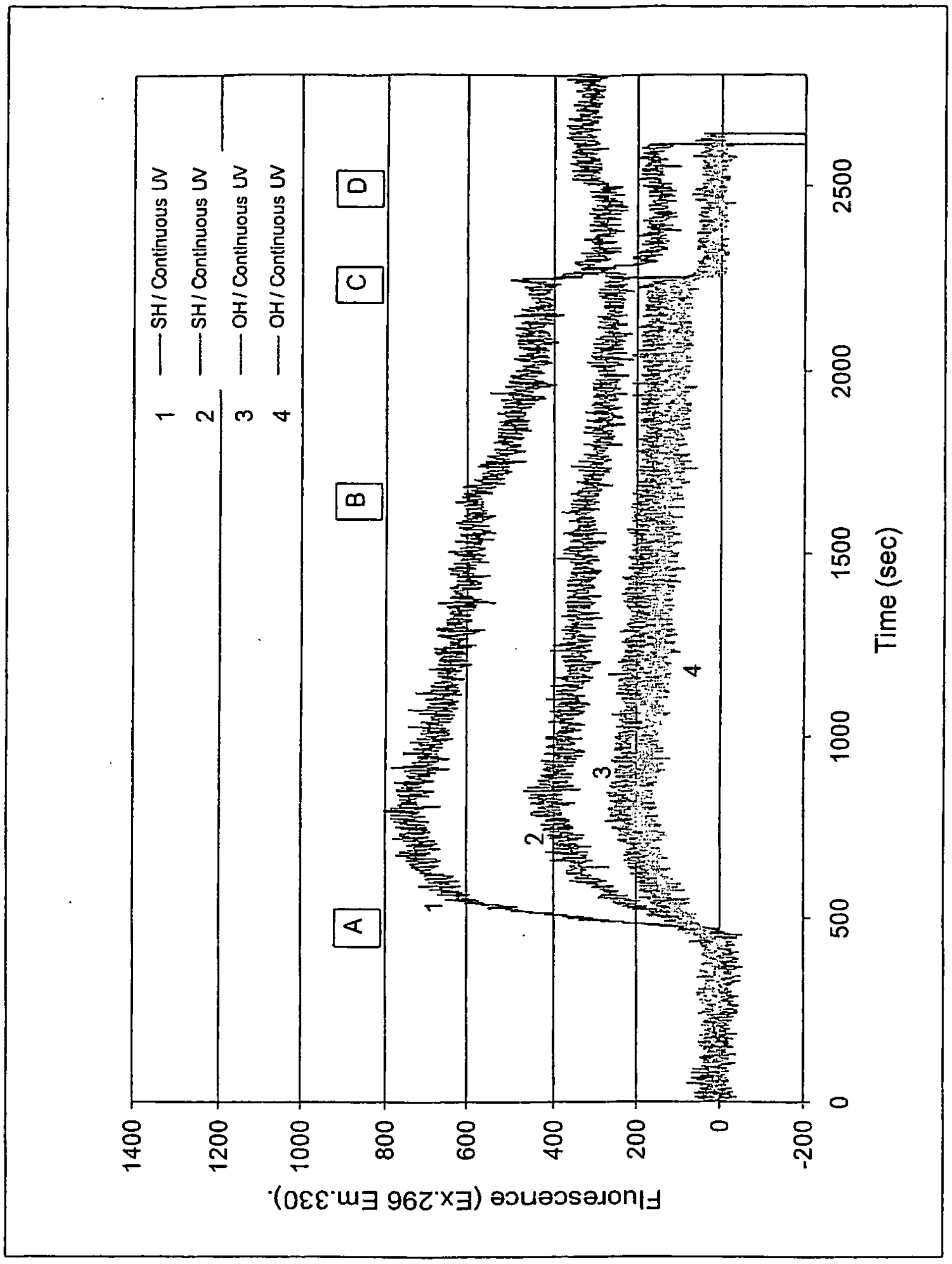
1 SH / Continuous UV
2 SH / Continuous UV
3 OH / Continuous UV
4 OH / Continuous UV
A
B
C
D
1
2
3
4
1400
1200
1000
800
600
400
200
0
-200
Fluorescence (Ex.296 Em.330).
0
500
1000
1500
2000
2500
Time (sec)

C

Figure 17 A

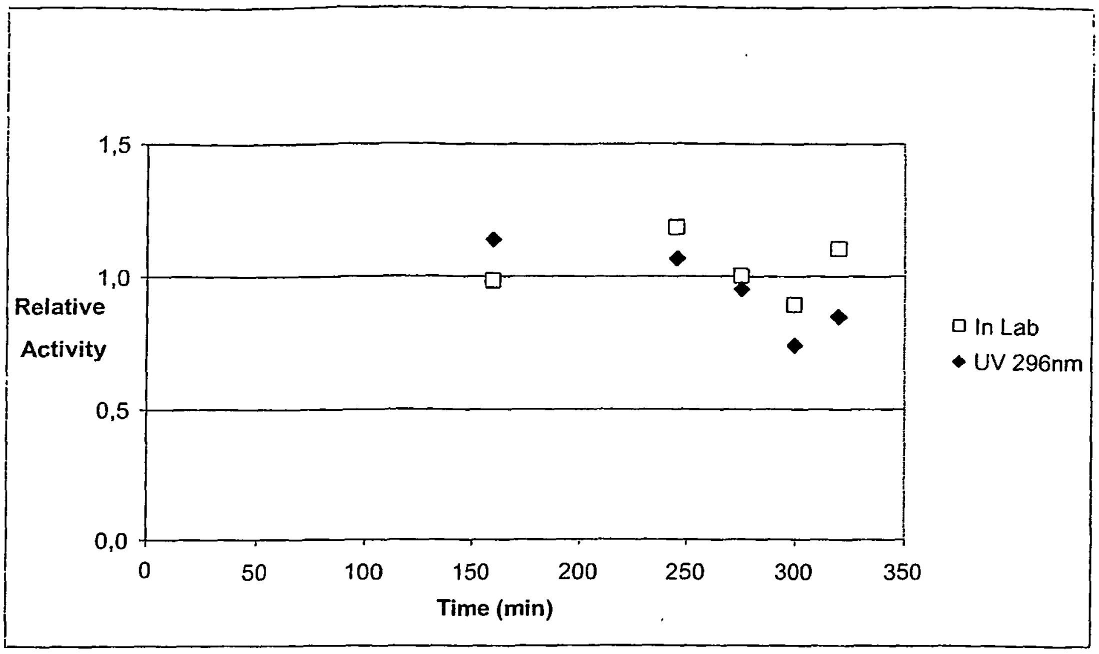


Figure 17B

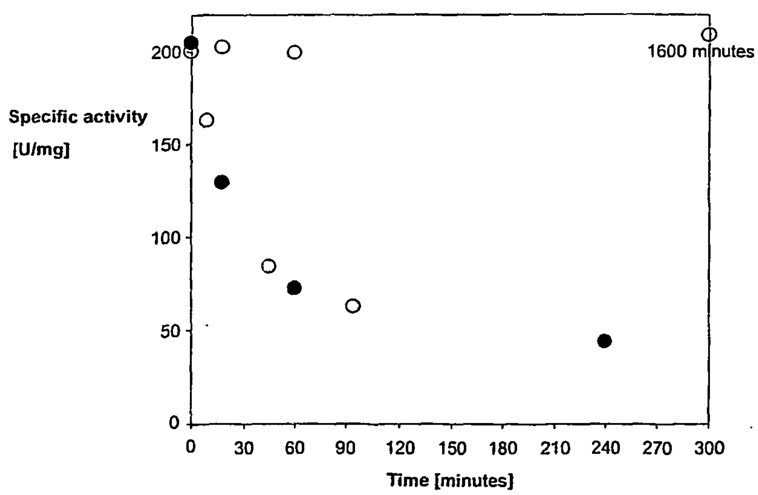

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6350368 B
- EP 0820483 A
- US 5412087 A
- US 6406844 B
- US 3959078 A
- WO 9116425 A
- WO 9401773 A


### Non-patent literature cited in the description


- **VEILLEUX J.** *IVD Technology,* March 1996, 26-31
- **WILSON D.S. ; NOCK S.** *Current Opinion in Chemical Biology,* 2001, vol. 6, 81-85
- **JOHNSSON B.** *Anal Biochem,* 1991, vol. 198, 268-77
- **ANDOLFI, L. et al.** *Arch. Biochem. Biophys.,* 2002, vol. 399, 81-88
- **SWEENEY, R.Y. et al.** *Anal Biochem.,* 2000, vol. 286, 312-314
- **COLLIOUD A et al.** *Bioconjugate Chem.,* 1993, vol. 4, 528-536
- **NEVES-PETERSEN MT. et al.** *Protein Science,* 2002, vol. 11, 588-600
- **PETERSEN MTN. et al.** *Protein Engineering,* 1999, vol. 12, 535-548
- **NEVES-PETERSEN MT et al.** *Protein Science,* 2002, vol. 11, 588-600
- **VANHOOREN A et al.** *Biochemistry,* 2002, vol. 41 (36), 11035-11043
- **SOLIDAY CL.** *Biochem Biophys Res Commun,* 1983, vol. 114, 1017-22
- **HONG Q.** *Biochemical Society Transactions,* 2001, vol. 29 (4), 587-582
- **ELLMAN GG.** *Arch. Biochem. Biophys.,* 1959, vol. 82, 70-77
- **HU ML.** *Meth. Enzymology,* 1994, vol. 233, 380-385
- **PROMPERS et al.** *FEBS Lett.,* 1999, vol. 456, 409-16
- **BERGMAN et al.** *Nucleic Acids Research,* 2000, vol. 28, 235-242
- **GUEX, N ; PEITSCH, M.C.** *Electrophoresis,* 1997, vol. 18, 2714-2723
- **MATHEWS, CHRISTOPHER K. ; VAN HOLDE, K. E.** Biochemistry. The Benjamin/Cummings Publishing Company, Inc, 1996
- **VANHOOREN et al.** *Biochemistry,* 2002, vol. 41 (36), 11035-11043
- **AYBAY et al.** *Immunology Letters,* 2003, vol. 85, 231-235